# EUROPEAN PATENT APPLICATION

(11) **EP 4 074 321 A1**
(43) Date of publication of application: **19.10.2022**
(21) Application number: 20899066.3
(22) Date of filing: 11.12.2020
(51) Int. Cl.: A61K 35/19, A61K 9/19, A61K 35/28, A61K 35/545, A61K 38/18, A61P 17/00, A61P 19/00, C12N 5/078, C12N 5/0789, C12N 5/10

(54) **FREEZE-DRIED PREPARATION CONTAINING MEGAKARYOCYTES AND PLATELETS**

(30) Priority: 12.12.2019 JP 2019224781
(71) Applicant: National University Corporation Chiba University, Chiba-shi, Chiba 263-8522 (JP); Kyoto University, Kyoto-shi, Kyoto 606-8501 (JP)
(72) Inventor: OHTORI, Seiji, Chiba-shi, Chiba 260-8670 (JP); SHIGA, Yasuhiro, Chiba-shi, Chiba 260-8670 (JP); KOSAKA, Kentaro, Chiba-shi, Chiba 260-8670 (JP); ETO, Koji, Kyoto-shi, Kyoto 606-8501 (JP)
(74) Representative: Hoffmann Eitle
(86) International application number: PCT/JP2020/046355
(87) International publication number: WO 2021/117886

(57) **Abstract**

The problem that differences among lots in components and amounts of growth factors and the like in platelet-rich plasmas derived from an autologous plasma and the freeze-dried preparations thereof are large, and the effects of the lots are not kept constant is solved.

Specifically, a freeze-dried preparation comprising megakaryocytes and platelets induced to differentiate from stem cells, a pharmaceutical composition comprising the freeze-dried preparation, and the like are provided.

## Description

### TECHNICAL FIELD

The present invention relates to a freeze-dried preparation comprising megakaryocytes and platelets. In particular, the present invention relates to a freeze-dried preparation comprising megakaryocytes and platelets, and use of the preparation for promoting a bone adhesion, or treating or preventing a skin disorder.

### BACKGROUND TECHNOLOGY

Factures due to an external wound and lumbar degenerative diseases such as lumbar spondylolisthesis and lumbar spinal canal stenosis are accompanied by troublesome symptoms such as a pain and paralysis associated with instability, and therefore any surgeries aimed at stabilizing the lesion is performed to fix the bone parts broken in continuity and the adjacent mobile elements. The stabilization by bone adhesion is a very important factor in these surgeries and is one of the major purposes of orthopedic surgical operations. However, as of now, the bone adhesion should be expected to occur in the natural course even after the surgery, which may take several weeks at the earliest and in some cases nearly a year to establish any complete bone adhesion, and, depending on the course, any pseud-arthrosis which is in a state of chronic instability without any complete bone adhesion may appear, causing a chronic instability and pain.

These courses of the healing for the bone adhesion sometimes lead to serious impairments of the patient's ADL (Activities of Daily Living) and QOL (Quality Of Life), and thus there is an urgent need in the field of orthopedics medical field to pursue the efficient promotion of the bone adhesion after surgeries.

Platelet Rich Plasma (PRP), which can be safely obtained by centrifuging and concentrating an autologous plasma, is drawing attention for this proposition. PRP, which is known to have a vigorous tissue repair potency, has been actively studied in the fields of plastic surgery, dentistry, and orthopedics, expecting the same to bring a repair effect on tendons and ligaments, and in the field of sports, the PRP is gathering attention as an autologous PRP therapy for major leaguers, thus further research on the PRP being underway worldwide. However, any PRP has not been vigorously studied to aim at the bone adhesion so far. The present inventors already demonstrated in the previous study that the PRP administration in the rat spinal fusion surgery significantly promoted the bone adhesion without any risk of infections or rejections (Non-Patent Document 1). Furthermore, the present inventors also reported that the combination of hydroxyapatite, which had been used as an artificial bone, and PRP was used to achieve a good bone adhesion in the animal experiment using rats (Non-Patent Document 2). Further, after obtaining those results in the animal experiments, the present inventors conducted two clinical trials using PRP as a translational research to obtain the result that the combination of the fresh PRP and the autologous bone or allogeneic bone in the spinal fusion surgery could reduce the bone adhesion period by an average of about 1-2 months in each case (Non-Patent Document 3). Since the PRP is highly safe and has an excellent bone adhesion-promoting effect as described above, the PRP should be very useful in the therapy of the orthopedic surgical field, thus suggesting strongly that the PRP could provide a promising therapeutic method.

In addition, the PRP can be used for PRP therapy. PRP therapy is a regenerative therapy in which the "healing power" inherent in humans is enhanced by utilizing the tissue repair potency of the proteins possessing a physiological activity, such as growth factors of platelets contained in autologous bloods. For example, the PRP can be administered to the affected areas in intractable cutaneous ulcers, decubitus (bedsores), burns, and necrosis or gangrene of the lower extremities in diabetic patients, as well as dental alveolar bone and gingival in order to promote the regenerations of the areas. Further, the PRP may be administered to patients to improve wrinkles on the skin of the face, neck and the like, and to promote hair growth. In particular, when administered to the skin, the PRP releases multiple proteins that possess a physiological activity, such as growth factors, increases the number of capillary lymph vessels in the skin, and makes skin fibroblasts migrate, thus expecting that PRP could regenerate the capillary lymph vessels or capillary blood vessels, and skin neogenesis granulation tissue.

However, the PRP has the drawback that the PRP should be prepared as an autologous PRP when used to effectively use the same by collecting a certain amount of the blood from the autologous patient immediately before use, since the PRP has an extremely short lifespan of several hours to several days. Specifically, when the PRP is used in surgery, it is necessary to collect about 200cc blood immediately before the surgery, which not only puts a heavy burden on the patient's body, but also requires a lot of manpower and efforts because of being prepared when used in a short time in addition to giving rise to difficulty in keeping the quality constant of the PRP. For these reasons, it has been extremely difficult to use the PRP especially in urgent situations such as an external wound. In order to overcome these drawbacks of such a fresh PRP, the present inventors have focused on a freeze-dried PRP (FD-PRP), and conducted the researches thereon. As a result, the present inventors confirmed that the most of the growth factors in the FD-PRP were maintained without disappearing even after the storage at room temperature for 8 weeks, and that the FD-PRP had the bone adhesion-promoting effect equivalent to that of a fresh PRP, demonstrating the effectiveness of the FD-PRP (Non-Patent Document 4, Non-Patent Document 5).

### CITATION LIST

### NON-PATENT DOCUMENTS

Non-Patent Document 1: Kamoda H, et al., J Bone Joint Surg Am. 2013 Jun 19; 95 (12): 1109-16
Non-Patent Document 2: Kamoda et al., Spine (Phila Pa 1976). 2012 Sep 15; 37 (20): 1727-33
Non-Patent Document 3: Kubota, et al. Asian Spine J. 2017 Apr; 11 (2): 272-277
Non-Patent Document 4: Shiga, et al. Sci Rep. 2016 Nov 11; 6: 36715
Non-Patent Document 5: Shiga Y et al., Asian Spine J., 2017, Jun; 11 (3): 329-336

### SUMMARY OF THE INVENTION

### PROBLEMS TO BE SOLVED BY THE INVENTION

In any case, from the viewpoint of further reduction of patient invasion and improvement of supply efficiency, it is considered that the safe preparations that are ready-made and commercialized would be more desirable than the both autologous blood-derived PRP and freeze-dried PRP which are prepared for each occasion when used by collecting the autologous blood. The PRP and freeze-dried PRP may be used in a large amount when administered to a wide range of the areas to be treated or to severe cases. However, it is difficult to facilitate a stable supply of the PRP because the platelets, which are the raw material of the PRP, have an extremely short expiration date of 4 days. At present, the platelets rely on a blood from a person's own or a donor, being concern that the supply will not be able to keep up with demand in many countries including Japan in the near future. In addition, there is also another problem that differences among lots in the components and the amounts of the growth factors and the like in the platelet-rich plasmas derived from an autologous plasma and the freeze-dried preparations thereof are large, and the effects of the lots are not kept constant.

Platelet production technology using induced pluripotent stem cells (iPSC) has been sought as a new platelet production system that does not depend on a person's own blood collection or a blood donation. Eto et al. disclosed in 2010 that platelets could be prepared from human iPS cells (Takayama, Eto, et al. J Exp Med. Vol. 207, December 20, 2010), and the same group also established in 2014 the method for producing the self-replicating megakaryocytes from human iPS cells, which provides them as the immortalized megakaryocyte cell line that could be freeze-stored in vitro (Nakamura, Eto, et al. Cell Stem Cell 14, 535-548, April 3, 2014). From June 2019, the clinical trials using the platelets as mentioned above have begun at Kyoto University for the first time in the world.

Under the circumstance, the question has been brought up whether or not a freeze-dried preparation prepared using the platelets induced to differentiate from pluripotent stem cells including iPS cells has a desired effect, for example the effect of promoting a bone adhesion without any side effects or adverse events, similarly to the PRP derived from the autologous plasmas and the freeze-dried PRP thereof.

### MEANS TO SOLVE PROBLEMS

The present inventors have earnestly studied, and, as a result, have confirmed that a freeze-dried preparation prepared using platelets induced to differentiate from stem cells in vitro should comprise growth factors in an abundant amount similarly to the PRP derived from autologous plasmas and the freeze-dried PRP thereof. The freeze-dried preparation should comprise megakaryocytes in addition to platelets, and be higher in the contents of BMP2 and BMP4 than those in the PRP derived from autologous plasmas and the freeze-dried PRP thereof. The present inventors examined the effect of the freeze-dried preparation to promote the bone adhesion in rats used as a lumbar posterior lateral fusion model, and found that the preparation should be vigorous in the bone adhesion and the bone formation potency, thereby completing the present invention.

Specifically, the present invention encompasses the following aspects.

### <Freeze-dried preparation>

[1] A freeze-dried preparation which comprises megakaryocytes and platelets.
[2] The freeze-dried preparation according to [1], wherein the megakaryocytes and the platelets are induced to differentiate from stem cells in vitro.
[3] The freeze-dried preparation according to [1] or [2], wherein the stem cells are pluripotent stem cells or hematopoietic stem cells.
[4] The freeze-dried preparation according to [2] or [3], wherein the pluripotent stem cells are iPS cells.
[5] The freeze-dried preparation according to any one of [1] to [4], which further comprises a growth factor released from the megakaryocytes and/or the platelets.
[6] The freeze-dried preparation according to [5], wherein the growth factor is at least one factor selected from the group consisting of bone morphogenetic proteins, platelet-derived growth factors, platelet-derived angiogenesis factors, transforming growth factors, vascular endothelial growth factors, epithelial growth factors, fibroblast growth factors, hepatocyte growth factors, platelet-derived endothelial cell growth factors, insulin-like growth factors, and platelet factor IV.

### <Pharmaceutical composition>

[7] A pharmaceutical composition for promoting a bone adhesion, or treating or preventing a skin disease, which comprises the freeze-dried preparation according to any one of [1] to [6].
[8] The pharmaceutical composition according to [7], which comprises the freeze-dried preparation according to any one of claims 1 to 6 in an amount effective for promoting a bone adhesion, or treating or preventing a skin disease.

### <Pharmaceutical use>

[9] A method for promoting a bone adhesion, or treating or preventing a skin disease, which comprises administering the freeze-dried preparation according to any one of claims 1 to 6 to a subject in need of such treatment.
[10] The method according to claim 9, which comprises administering the freeze-dried preparation according to any one of claims 1 to 6 in an amount effective for promoting a bone adhesion, or treating or preventing a skin disease to a subject in need of such treatment.
[11] The freeze-dried preparation according to any one of [1] to [6] for promoting a bone adhesion, or treating or preventing a skin disease.
[12] Use of the freeze-dried preparation according to any one of [1] to [6] for manufacturing a medicament for promoting a bone adhesion, or treating or preventing a skin disease.

### <Method for producing freeze-dried preparations>

[13] A method for producing a freeze-dried preparation comprising megakaryocytes and platelets, which is characterized in:
   (A) preparing a cell mixture of platelets and megakaryocytes, and
   (B) freeze-drying the cell mixture of the platelets and the megakaryocytes.
[14] The method according to [13], wherein the megakaryocytes are immortalized megakaryocytes that are induced to differentiate from stem cells in step (A).
[15] The method according to [13] or [14], wherein the cell mixture prepared in step (A) is activated.

### EFFECT OF INVENTION

According to the present invention, PRPs derived from platelets induced to differentiate from stem cells including iPS cells are freeze-dried, thereby making it possible to provide a pharmaceutical medicament when necessary. If FD-PRPs derived from iPS cells free of antigenicity are developed and can be safely used for allogeneic patients, the need for preparation when used in surgery would be eliminated, thus dramatically increasing in the possibility of their general usefulness as effective and new medicaments. In addition, if ready-made and commercialized materials effectively promote a fast bone adhesion in fractures and spinal surgery without any patient invasion or burden on the medical staff, a dramatic improvement in patient's ADL and QOL may be achieved, expecting that it would greatly contribute to the improvement of the medical economy by reducing and alleviating complications due to surgery, and shortening the length of hospital stay.

### BRIEF DESCRIPTION OF DRAWINGS

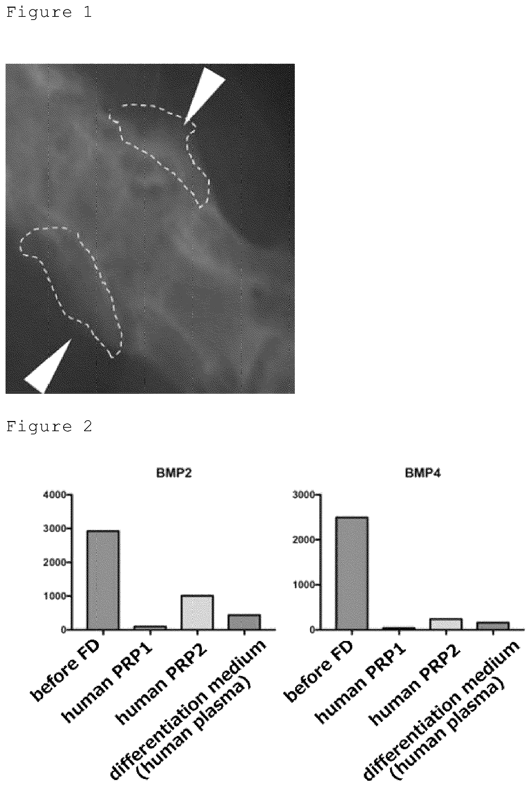

Figure 1 is an X-ray image of a rat spine showing that the freeze-dried preparation of the present invention comprising iPS cells-derived immortalized megakaryocytes and platelets has an excellent bone adhesion-promoting effect and bone-forming potency in a lumbar fusion rat model.
Figure 2 represents graphs showing the measurement results of the BMP2 and BMP4 concentrations in the preparation immediately after the activation of megakaryocytes/platelets, i.e., before freeze-drying (before FD) in the preparation process of the freeze-dried preparation of the present invention, in the preparation in which PRPs as prepared from human peripheral blood comprising the same number of the platelets were similarly activated (human PRP1, human PRP2), and in the control (differentiation medium). It is shown that the preparation of the present invention (before FD) are higher in the BMP2 and BMP4 concentrations than those in the activated PRPs comprising the same number of platelets.

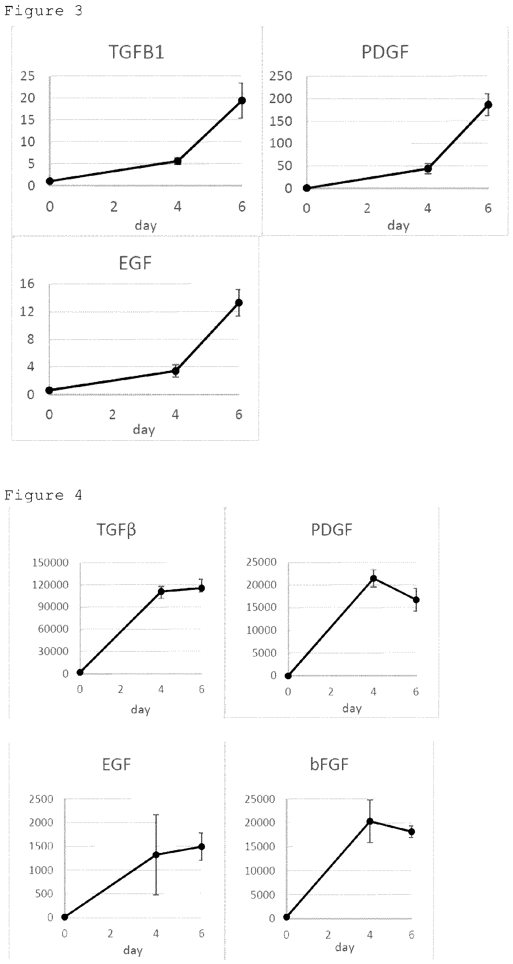

Figure 3 represents graphs showing the changes in cytokine gene expressions of the mature megakaryocytes and platelets (MMK-PLT) derived from the iPS cell-derived immortalized megakaryocyte cell lines (imMKCL) in accordance with their maturation.
Figure 4 represents graphs showing the changes in the amounts of cytokine proteins (pg/ml) released from the activated mature megakaryocytes and platelets (MMK-PLT) derived from the iPS cell-derived immortalized megakaryocyte cell lines (imMKCL) in accordance with their maturation.

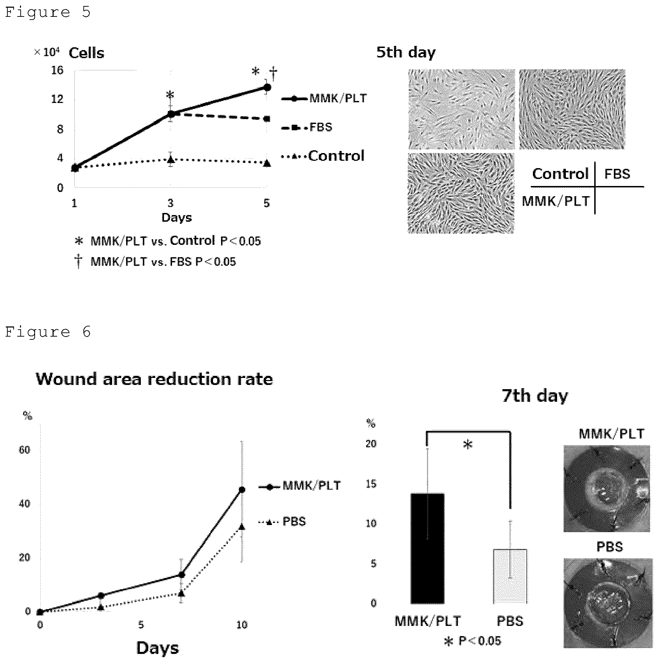

Figure 5 (the left) is a graph showing the effects of the supernatant obtained by activating the mature megakaryocytes and platelets (MMK/PLT) derived from the iPS cell-derived immortalized megakaryocyte cell lines (imMKCL) on the proliferation of human primary culture fibroblasts. Figure 5 (the right) is a photograph of the human primary culture fibroblasts on the 5th day of culture.
Figure 6 (the left) is a graph showing the effects of the mature megakaryocytes and platelets (MMK/PLT) derived from the iPS cell-derived immortalized megakaryocyte cell lines (imMKCL) on the wound area reduction rates. Figure 6 (the right) is a graph of the wound area reduction rates and a photograph of the wound on the 7th day.

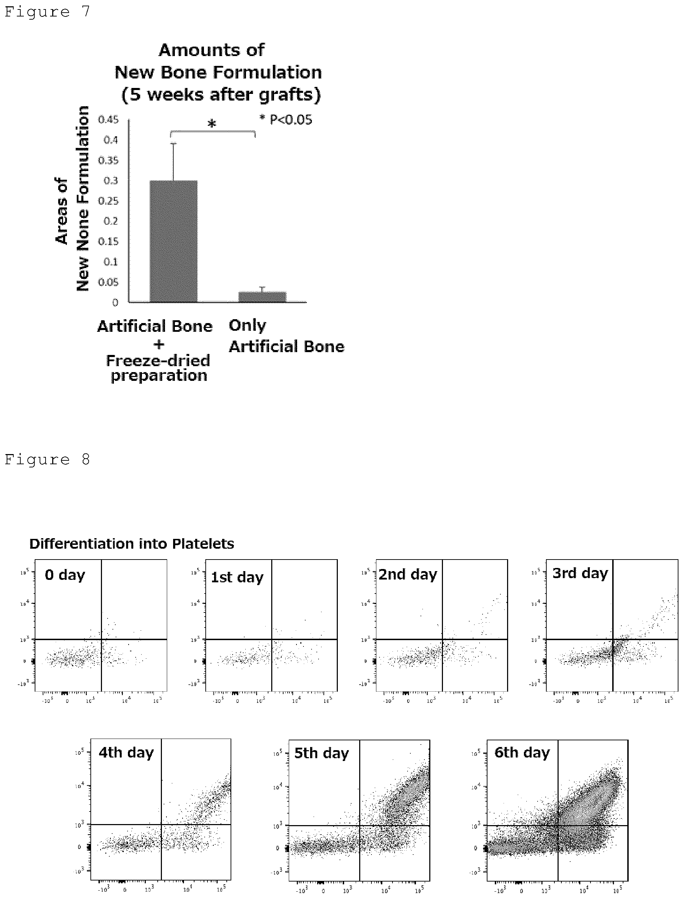

Figure 7 is a graph showing the effect of the freeze-dried preparation comprising the megakaryocytes and platelets derived from the iPS cell-derived immortalized megakaryocyte cell lines on the new bone formation in a rat lumbar fusion model.
Figure 8 shows the results of the differentiations of the iPS cell-derived immortalized megakaryocyte cell lines into the platelets as monitored over time with a flow cytometer. The upper right population corresponds to the platelets. The vertical axis shows the side scattering, and the horizontal axis shows the forward scattering.

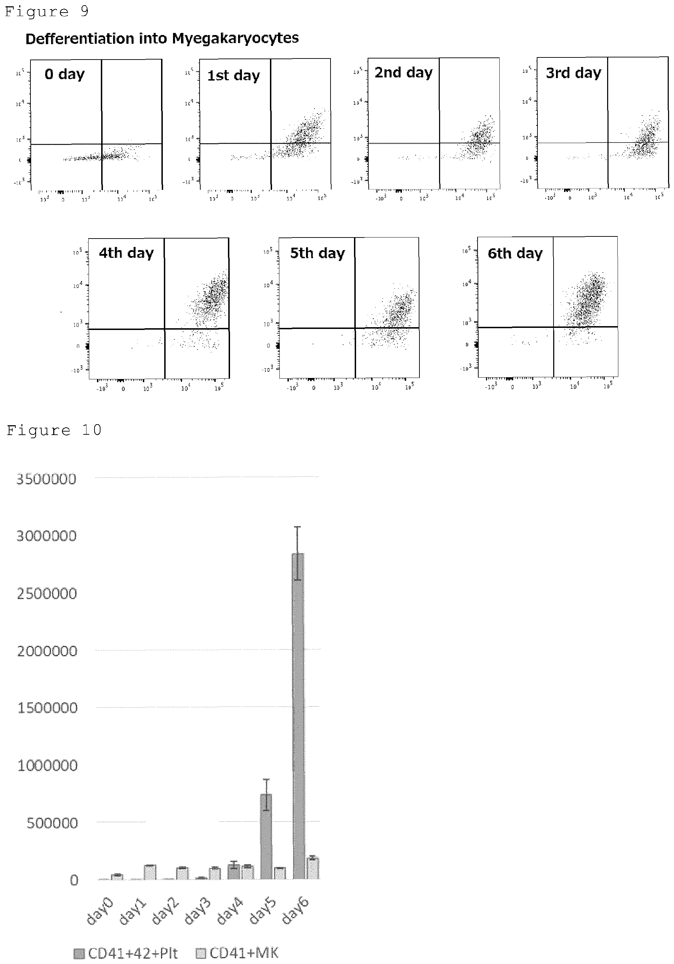

Figure 9 shows the differentiations of the iPS cell-derived immortalized megakaryocyte cell lines into the mature megakaryocytes over time with a flow cytometer. The upper right population corresponds to the mature megakaryocytes. The vertical axis shows the side scattering, and the horizontal axis shows the forward scattering.
Figure 10 is a graph showing the changes over time in the numbers of the platelets (CD41+42+Plt) and the mature megakaryocytes (CD41+MK) after the differentiation and culture.

### EMBODIMENTS FOR CARRYING OUT INVENTION

### <Freeze-dried preparations>

In an embodiment, the present invention relates to a freeze-dried preparation comprising megakaryocytes and platelets.

The freeze-dried preparations of the present invention have a tissue repair potency. Similarly, platelet-rich plasma (PRP) derived from blood, and/or freeze-dried PRP preparations obtained by lyophilizing the same, which have been widely confirmed in the fields of plastic surgery, dentistry and orthopedics to have a tissue repair potency, have been known (Kamoda H, et al., J Bone Joint Surg Am. 2013 Jun 19; 95 (12): 1109-16, Shiga, et al. Sci Rep. 2016 Nov 11; 6: 36715, Shiga Y, Asian Spine J. 2017 Jun; 11 (3): 329-336.). Since those PRP preparations do not substantially comprise any "megakaryocytes", they are different from the freeze-dried preparations of the present invention in this respect. In particular, the freeze-dried preparations of the present invention differ from the conventional PRP preparations in that the former comprise the megakaryocytes in a relatively large amount. The freeze-dried preparations of the present invention may also differ in that they have a higher content of the certain humoral factors such as BMP2 and/or BMP4 as compared to the conventional PRP preparations. Megakaryocytes, which are derived from hematopoietic stem cells in a living body, maturate in the bone marrow via megakaryocyte progenitor cells (CFU-Meg), megakaryoblasts, and promegakaryocytes, and they are not divided with only the nucleus therein dividing from 2N to 64N, thereby being the result of maturation into large cells with a large polymorphonucleus. Megakaryocytes, which are generally present near sinusoidal blood vessels in the bone marrow, shall not leave the bone marrow, and therefore may not be observed in peripheral blood. They undergo intracellular nuclear divisions without cell divisions and have a polymorphonucleus. One megakaryocyte produces thousands of platelets. Since platelets are cells essential for thrombus formation and hemostasis, the demand for platelets is extremely high in treatment for leukemia, bone marrow transplantation and thrombocytopenia, anticancer therapy, and the like.

In the present invention, "megakaryocytes" mean any megakaryocytes capable of providing platelets, and include immortalized megakaryocytes, immature megakaryocytes, mature megakaryocytes, and the like. In the present invention, "immature megakaryocytes" are defined as megakaryocytes whose polyploidization has not proceeded (about 2N to 8N), and include for example megakaryocytes that express the genes related to megakaryocyte maturation (GATA1, NF-E2, c-MPL, β1-tubulin and MYH9) in a low level and MYH10 in a high level among the megakaryocytes expressing CD41. Therefore, immature megakaryocytes can be reworded as "megakaryocyte progenitor cells". Examples of such immature megakaryocytes include immature megakaryocytes artificially prepared from hematopoietic progenitor cells. In the present invention, the immature megakaryocytes may or may not be cloned, and the cloned one may be referred to as immature megakaryocyte cell lines, without especially limiting. On the other hand, "mature megakaryocytes" are defined as megakaryocytes whose polyploidization has proceeded (8N or more), which express the genes related to megakaryocyte maturation in a high level and MYH10 in a low level. In the present invention, "maturation" means that the maturation of megakaryocytes proceeds, namely, that the polyploidization or the increase in cytoplasm proceeds, that the expression levels of the genes related to megakaryocyte maturation are increased, and that the expression levels of the genes related to megakaryocyte immaturity are decreased, and they encompass any cells which do not necessarily reach the mature megakaryocytes as defined above as long as those cells show any tendency of the maturation as compared with cells before the maturation steps. In addition, "maturation" refers to the case in which, when the maturations of the cell populations before and after culture are compared, the maturation of the latter population as a whole is proceeding, namely, the case in which the total number of nuclei in the whole cell population is increased, or (in addition) the cytoplasm is increased (or the volume is increased).

In the present invention, "freeze-dried preparation" is defined as a preparation in the form of powder or granule as prepared by a process such as a freeze-drying or lyophilization well known to those skilled in the art. The freeze-dried preparations are chemically and biologically stable, and can be stored for a long period of time. When using the freeze-dried preparations, they are dissolved or suspended in a water or physiological saline at the time of administration to the subject.

A specific embodiment of the present invention relates to a freeze-dried preparation of the present invention comprising megakaryocytes and platelets in which the megakaryocytes and platelets are induced to differentiate from stem cells in vitro, more specifically, to a freeze-dried preparation of the present invention in which the stem cells are pluripotent stem cells or hematopoietic stem cells, and more specifically, to a freeze-dried preparation of the present invention in which the pluripotent stem cells are iPS cells.

In the present invention, "stem cell" means a cell having both differentiation/pluripotency that makes it capable to differentiate into many types of cells that constitute a living body, and self-renewal competence that makes it capable to maintain differentiation/pluripotency even after the division and proliferation, and includes any stem cell that can differentiate into platelets.

In the present invention, "pluripotent stem cell" means a stem cell that does not form any individuals but have a potency to differentiate into all cell lineages belonging to three germ layers (endoderm, mesoderm, ectoderm), and includes any pluripotent stem cell that can differentiate into platelets. Preferred examples of pluripotent stem cells are ES cells, iPS cells, embryonic germ stem cells (EG cells) derived from primordial germ cells, embryonic stem (ntES) cells derived from cloned embryos obtained by nuclear transplantation, spermatogonial stem cells (GS cells), pluripotent cells derived from cultured fibroblasts or bone marrow stem cells (Muse cells), and the like. The ES cells may be ES cells generated by nuclear reprogramming from somatic cells. The pluripotent stem cells are preferably the ES cells or the iPS cells. The pluripotent stem cells may be of mammalian origin, and preferably are human-derived pluripotent stem cells.

Hematopoietic stem cells are defined as stem cells that can differentiate into hematopoietic cells, and in a human adult, they are mainly present in the bone marrow, and generate leukocytes (neutrophils, eosinophils, basophils, lymphocytes, monocytes, and macrophages), red blood cells, platelets, mast cells, and dendritic cells. Hematopoietic stem cells are also referred to as hematocytoblast or bone marrow stem cells.

Hereinafter, preferred examples of the pluripotent stem cells are described.

ES cells are defined as pluripotent stem cells derived from an embryo at an early stage in the development of animals called the blastocyst stage. The ES cells can be established by taking out the inner cell mass from the blastocysts of fertilized eggs of a mammalian animal and culturing the inner cell mass on fibroblasts as feeder cells. The maintenance of the cells by subculture can be carried out using a culture medium supplemented with the substances such as leukemia inhibitory factor (LIF) and basic fibroblast growth factor (bFGF). Methods for establishing and maintaining human and monkey ES cells are described in, for example, USP5,843,780; Thomson JA, et al. (1995), Proc Natl. Acad. Sci. U S A. 92:7844-7848; Thomson JA, et al. (1998), Science. 282:1145-1147; H. Suemori et al. (2006), Biochem. Biophys. Res. Commun., 345:926-932; M. Ueno et al. (2006), Proc. Natl. Acad. Sci. USA, 103:9554-9559; H. Suemori et al. (2001), Dev. Dyn., 222:273-279; H. Kawasaki et al. (2002), Proc. Natl. Acad. Sci. USA, 99: 1580-1585; Klimanskaya I, et al. (2006), Nature. 444:481-485, and so on.

The ES cells can be typically selected using the expression of gene markers such as alkaline phosphatase, Oct-3/4, and Nanog, as an index. In particular, the human ES cells can be selected by detecting the expression of gene markers such as OCT-3/4, NANOG, etc., by a real-time PCR method and/or detecting SSEA-3, SSEA-4, TRA-1-60, and TRA-1-81 as cell surface antigens by an immunostaining method (Klimanskaya I, et al. (2006), Nature, 444: 481-485) .

Various mouse ES cell lines established by inGenious, RIKEN (RIKEN), etc., can be used as mouse ES cells. Various human ES cell lines established by the National Institutes of Health (NIH), RIKEN, Kyoto University, and Cellartis can be used as human ES cells. For example, CHB-1 to CHB-12 cell lines, RUES1 cell line, RUES2 cell line, HUES1 to HUES28 cell lines, etc. from NIH, WA01 (H1) cell line, WA09 (H9) cell line from WisCell Research Institute, and KhES-1 cell line, KhES-2 cell line, KhES-3 cell line, KhES-4 cell line, KhES-5 cell line, SSES1 cell line, SSES2 cell line, SSES3 cell line, etc. from RIKEN are available as mouse ES cells. As human ES cell lines, for example, WA01 (H1) and WA09 (H9) are available from the WiCell Research Institute, and KhES-1, KhES-2, KhES-3 and KthES11 are available from the Institute for Frontier Life and Medical Sciences, Kyoto University (Kyoto, Japan).

IPS cells, which are also referred to as artificial pluripotent stem cells or induced pluripotent stem cells, are cells having acquired differentiation/pluripotency and self-renewal competence, which can be prepared either by introducing one or more particular nuclear reprogramming substances into somatic cells such as fibroblasts in the form of DNA or protein, or increasing the expression levels of the endogenous mRNAs and proteins of the nuclear reprogramming substances by the use of one or more particular agents (K. Takahashi and S. Yamanaka (2006) Cell, 126:663-676; K. Takahashi et al. (2007), Cell, 131:861-872; J. Yu et al. (2007), Science, 318:1917-1920; Nakagawa, M. 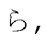 , Nat. Biotechnol. 26:101-106 (2008); International Publication WO 2007/069666). As used herein, "somatic cell" means any animal cell (preferably a mammalian cell including human) other than germ-line cells such as eggs, egg mother cells, and ES cells, or cells having totipotency, and includes somatic cells of embryos (fetal), somatic cells of newborn babies, and somatic cells of mature healthy bodies or diseased somatic cells, as well as any primary cultured cells, passaged cells, and established cells. Particular examples of the somatic cells include, for example, (1) tissue stem cells (somatic stem cells) such as a neural stem cell, a hematopoietic stem cell, a mesenchymal stem cell, and a dental pulp stem cells, (2) tissue progenitor cells, (3) differentiated cells such as a lymphocyte, an epithelial cell, an endothelial cell, a muscle cell, a fibroblast (a skin cell, etc.), a hair cell, a hepatocyte, a gastric mucosal cell, an intestinal cell, a splenocyte, a pancreatic cell (a pancreatic exocrine cell, etc.), a brain cell, a lung cell, a renal cell, and a fat cell.

The nuclear reprogramming substance may be selected from a gene specifically expressed in an ES cell, a gene product or non-cording RNA thereof, or a gene playing an important role in maintaining the undifferentiated state of an ES cell, or a gene products or non-coding RNA thereof, and a low molecular weight compound. Examples thereof include, but not particularly limited to, for example, Oct3/4, Klf4, Klf1, Klf2, Klf5, Sox2, Sox1, Sox3, Sox15, Sox17, Sox18, c-Myc, L-Myc, N-Myc, TERT, SV40LargeTantigen, HPV16E6, HPV16E7, Bmil, Lin28, Lin28b, Nanog, Fbx15, ERas, ECAT15-2, Tcl1, beta-catenin, Sall1, Sall4, Esrrb, Esrrg, Nr5a2, Tbx3 and Glis1. When establishing an iPS cell, these reprogramming substances may be used alone or in combination. Such combination of the reprogramming substances may include at least one, two or three types of the reprogramming substance, and preferably include three or four types of the reprogramming substance.

The information of the nucleotide sequences of mouse and human cDNAs of each of the nuclear reprogramming substances as described above and the amino acid sequences of proteins encoded by the cDNAs can be found by referring to GenBank (US NCBI) or the EMBL (Germany) accession numbers described in WO2007/069666. In addition, the information of the mice and human cDNA sequences and amino acid sequences of L-Myc, Lin28, Lin28b, Esrrb, Esrrg, and Glis1 can be obtained by referring to the NCBI accession numbers shown in Table 1. Those skilled in the art can prepare desired nuclear reprogramming substances by conventional methods, based on the information of the cDNA sequences or amino acid sequences.

**Table 1**

| Gene Name | Mouse | Human |
|---|---|---|
| **L-Myc** | **NM_008506** | **NM_001033081** |
| **Lin28** | **NM_145833** | **NM_024674** |
| **Lin28b** | **NM_001031772** | **NM_001004317** |
| **Esrrb** | **NM_011934** | **NM_004452** |
| **Esrrg** | **NM_011935** | **NM_001438** |
| **Glis1** | **NM_147221** | **NM_147193** |

These nuclear reprogramming substances may be each introduced in the form of a protein into a somatic cell by techniques such as lipofection, binding to a cell membrane-permeable peptide or microinjection, or may be introduced in the form of DNA into a somatic cell by techniques such as using a vector, for example, virus, plasmid, or artificial chromosome vector, lipofection, the use of liposome, or microinjection. Examples of the virus vector include retroviral vectors, lentivirus vectors (both are described in Cell, 126, pp.663-676, 2006; Cell, 131, pp.861-872, 2007; Science, 318, pp.1917-1920, 2007), adenovirus vectors (Science, 322, 945-949, 2008), adeno-associated viral vectors, and Sendai virus vectors (Proc Jpn Acad Ser B Phys Biol Sci. 85, 348-62, 2009). Further, examples of the artificial chromosome vector include, for example, a human artificial chromosome (HAC), a yeast artificial chromosome (YAC), and bacterial artificial chromosomes (BAC, and PAC). As the plasmid, a plasmid for mammalian cells can be used (Science, 322: 949-953, 2008). The vectors can contain control sequences such as a promoter, an enhancer, a ribosomal binding sequence, a terminator, and a polyadenylation site or a polyadenylation signal so that nuclear reprogramming substances can be expressed. Examples of the promoter to be used include, for example, EF1α promoter, CAG promoter, SRα promoter, SV40 promoter, LRT promoter, CMV (cytomegalovirus) promoter, RSV (Rous sarcoma virus) promoter, MoMuLV (Moloney murine leukemia virus) LTR, and HSV-TK (herpes simplex virus thymidine kinase) promoter. Among others, EF1α promoter, CAG promoter, MoMuLV LTR, CMV promoter, and SRα promoter are preferable. In addition, the vectors may contain a selection marker sequence such as a drug resistance gene (for example, a kanamycin-resistant gene, an ampicillin-resistant gene, or a puromycin-resistant gene), a thymidine kinase gene, and a diphtheria toxin gene of a fragment thereof; a reporter gene sequence such as a green fluorescence protein (GFP), β-glucuronidase (GUS), or FLAG; and the like, if necessary. In order to remove a gene encoding a nuclear reprogramming substance or both of a promoter and the gene encoding a nuclear reprogramming substance binding thereto after the introduction into a somatic cell, the vectors may also have LoxP sequences thereacross. In another preferable embodiment, after incorporating a transgene into a chromosome by using a transposon, a method can be used which involves completely removing the transgene from the chromosome by causing a transferase to act on the cell using a plasmid vector or an adenovirus vector. Preferred examples of the transposon include, for example, piggyBac as a transposon derived from a lepidopteran insect (Kaji, K. et al., (2009), Nature, 458:771-775, Woltjen et al., (2009), Nature, 458: 766-770, WO 2010/012077). Further, the vectors may contain the origin of lymphotrophic herpes virus, BK virus, or Bovine papillomavirus, and a sequence responsible for the replication thereof so that they are replicated even without incorporating them into a chromosome or are episomally present. Examples therefor include the involvement of EBNA-1 and oriP sequences, or Large T and SV40ori sequences (WO2009/115295, WO2009/157201, and WO2009/149233). An expression vector for polycistronic expression may be used to simultaneously introduce two or more nuclear reprogramming substances. For polycistronic expression, gene-encoding sequences may be bound to each other through IRES or a foot-and-mouth disease virus (FMDV) 2A coding region (Science, 322: 949-953, 2008, WO2009/092042 and WO2009/152529).

To increase the efficiency of the induction of an iPS cell in nuclear reprogramming, in addition to the above factors, for example, a histone deacetylase (HDAC) inhibitor [for example, a small molecule inhibitor, such as valproic acid (VPA) (Nat. Biotechnol., 26(7): 795-797 (2008)), trichostatin A, sodium butyrate, MC1293, or M344, a nucleic acid expression inhibitor, such as siRNA or shRNA against HDAC (for example, HDAC1 siRNA Smartpool^{®} (Millipore)) or HuSH 29mer shRNA Constructs against HDAC1 (OriGene)], a DNA methyltransferase inhibitor (for example, 5'-azacytidine) (Nat. Biotechnol., 26(7): 795-797 (2008)), a G9a histone methyltransferase inhibitor [for example, a small molecular inhibitor, such as BIX-01294 (Cell Stem Cell, 2: 525-528 (2008)), or a nucleic acid expression inhibitor, such as siRNA or shRNA against G9a (for example, G9a siRNA (human) (Santa Cruz Biotechnology)], an L-channel calcium agonist (for example, Bayk8644) (Cell Stem Cell, 3, 568-574 (2008)), a p53 inhibitor (for example, an siRNA and shRNA against p53) (Cell Stem Cell, 3, 475-479 (2008)), a Wnt signaling activator (for example, soluble Wnt3a) (Cell Stem Cell, 3, 132-135 (2008)), a growth factor such as LIF or bFGF, an ALK5 inhibitor (for example, SB431542) (Nat. Methods, 6: 805-8 (2009)), a mitogen-activated protein kinase signaling inhibitor, a glycogen synthase kinase-3 inhibitor (PloS Biology, 6(10), 2237-2247 (2008)), and a miRNA, such as miR-291-3p, miR-294, or miR-295 (R. L. Judson et al., Nat. Biotech., 27: 459-461 (2009)), can be used.

Agents are used to increase the expression of an endogenous protein of a nuclear reprogramming substance. Examples of such agents include 6-bromoindirubin-3'-oxime, indirubin-5-nitro-3'-oxime, valproic acid, 2-(3-(6-methylpyridin-2-yl)-1H-pyrazol-4-yl)-1,5-naphthyridine, 1-(4-methylphenyl)-2-(4,5,6,7-tetrahydro-2-imino-3(2H)-benzothiazolyl)ethane HBr (pifithrin-alpha), prostaglandins (for example, prostaglandin J2, and prostaglandin E2 (WO2010/068955).

Further, for the purpose of increasing the establishment efficiency, an ARID3A inhibitor (for example, an siRNA and shRNA for ARID3A), neuropeptide Y, UTF1, IRX6, PITX2, DMRTBl and the like may be used.

Examples of the combination of a nuclear reprogramming substance include the combinations described in WO2007/096666, WO2008/118820, WO2009/007852, WO2009/032194, WO2009/058413, WO2009/057831, WO2009/075119, WO2009/079007, WO2009/091659, WO2009/101084, WO2009/101407, WO2009/102983, WO2009/114949, WO2009/117439, WO2009/126250, WO2009/126251, WO2009/126655, WO2009/157593, WO2010/00915, WO2010/033906, WO2010/033920, WO2010/042800, WO2010/050626, WO 2010/056831, WO2010/068955, WO2010/098419, WO2010/102267, WO 2010/111409, WO 2010/111422, WO2010/115050, WO2010/124290, WO2010/147395, WO2010/147612, Huangfu D, et al. (2008), Nat. Biotechnol., 26: 795-797, Shi Y, et al. (2008), Cell Stem Cell, 2: 525-528, Eminli S, et al. (2008), Stem Cells. 26: 2467-2474, Huangfu D, et al. (2008), Nat Biotechnol. 26:1269-1275, Shi Y, et al. (2008), Cell Stem Cell, 3, 568-574, Zhao Y, et al. (2008), Cell Stem Cell, 3: 475-479, Marson A, (2008), Cell Stem Cell, 3, 132-135, Feng B, et al. (2009), Nat Cell Biol. 11: 197-203, R.L. Judson et al., (2009), Nat. Biotech., 27: 459-461, Lyssiotis CA, et al. (2009), Proc Natl Acad Sci U S A. 106: 8912-8917, Kim JB, et al. (2009), Nature. 461: 649-643, Ichida JK, et al. (2009), Cell Stem Cell. 5: 491-503, Heng JC, et al. (2010), Cell Stem Cell. 6:167-74, Han J, et al. (2010), Nature. 463:1096-100, Mali P, et al. (2010), Stem Cells. 28:713-720, Maekawa M, et al. (2011), Nature. 474: 225-9.

Culture media for inducing iPS cells include, for example, (1) 10 to 15% FBS-containing DMEM, DMEM/F12, or DME medium (these media may further properly contain LIF, penicillin/streptomycin, puromycin, L-glutamine, a non-essential amino acid, β-mercaptoethanol, and the like), and (2) a medium for culturing ES cells containing bFGF or SCF, for example, a medium for culturing mouse ES cells (for example, TX-WES medium (Thrombo X) or a medium for culturing primate ES cells (for example, a medium for primate (human and monkey) ES cells (ReproCell, Kyoto, Japan), mTeSR-1).

As an example for a culture method, for example, somatic cells can be contacted with a nuclear reprogramming substance (DNA, RNA, or protein) in a 10% FBS-containing DMEM or DMEM/F12 medium at 37°C in the presence of 5% CO₂, and cultured for about 4 to 7 days, followed by reseeding the cells on feeder cells (for example, mitomycin C-treated STO cells or SNL cells), and culturing the cells in a bFGF-containing medium for culturing primate ES cells from about 10 days after the contact between the somatic cells and the nuclear reprogramming substance, thereby generating ES cell-like colonies about 30 to about 45 days or more after the contact. To increase the efficiency of the induction of iPS cells, the culture may also be performed under conditions of an oxygen concentration as low as 5 to 10%.

Alternatively, the cells may be cultured in a 10% FBS-containing DMEM medium (this may appropriately contain further LIF, penicillin/streptomycin, puromycin, L-glutamine, a non-essential amino acid, b-mercaptoethanol, and the like) on feeder cells (for example, mitomycin C-treated STO cells or SNL cells, and the like) at 37°C in the presence of 5% CO₂, to form ES-like colonies about 25 to about 30 days or more after the culture. An alternative method therefor may be also exemplified, in which the reprogrammed somatic cells themselves (Takahashi K, et al. (2009), PLoS One. 4: E8067 or WO2010/137746), or extracellular matrixes (for example, Laminin-5 (WO2009/123349) and Matrigel (BD)) are used instead of the feeder cells.

In addition, a method for culture using a serum-free medium is also exemplified (Sun N, et al. (2009), Proc Natl Acad Sci USA. 106: 15720-15725). Further, in order to increase the establishment efficiency, iPS cells may be established under hypoxic conditions (an oxygen concentration of 0.1% or more and 15% or less) (Yoshida Y, et al. (2009), Cell Stem Cell. 5: 237-241 or WO2010/013845).

During the above culture, the medium is replaced with a fresh medium once daily from at or after the second day of starting the culture. The number of the somatic cells used in nuclear reprogramming is not limited, but it may be in the range of about 5 × 10³ to about 5 × 10⁶ per culture dish (100 cm²).

IPS cells can be selected according to the shape of the formed colonies. When a DNA that contains a drug-resistant gene expressed in association with a gene expressed on the case that a somatic cell is reprogramed (for example, Oct3/4, Nanog) is used as a marker gene, marker gene-expressing cells (i.e., established iPS cells) can be selected by performing culture in a medium containing the corresponding drug (i.e., a selection medium). Marker gene-expressing cells (i.e., established iPS cells) can also be detected or selected by observation under a fluorescence microscope when the marker gene is a fluorescent protein gene, by adding a light-emitting substrate when the marker gene is a luciferase gene, or by adding a chromogenic substrate when the marker gene is a chromogenic enzyme gene.

As used herein, "somatic cell" may be any cell other than a germ cell including a cell derived from a mammal (for example, a human, a mouse, a monkey, a pig, or a rat). Examples of the somatic cell include, but are limited to, somatic cells of embryos (fetal), somatic cells of newborn babies, and somatic cells of mature healthy bodies or diseased somatic cells, as well as any primary cultured cells, passaged cells, and established cells. Particular examples of the somatic cells include, for example, keratinized epithelial cells (e.g., a keratinized epidermal cell), mucosal epithelial cells (e.g., an epithelial cell of the tongue surface), exocrine gland epithelial cells (e.g., a mammary gland cell), hormone-secreting cells (e.g., an adrenomedullary cell), cells for metabolism/storage (e.g., a liver cell), luminal epithelial cells forming the interface (e.g., a type I alveolar cell), luminal epithelial cells of an inner chain tube (e.g., a vascular endothelial cell), ciliated cells having transport capacity (e.g., a tracheal epithelial cell), cells for extracellular matrix secretion (e.g., a fibroblast), constrictive cells (e.g., a smooth muscle cell), cells of the blood and the immune system (e.g., a T-lymphocyte), sense-related cells (e.g., a rod cell), autonomic neurons (e.g., a cholinergic neuron), sustentacular cells of sensory organs and peripheral neurons (e.g., a satellite cell), neurons and glial cells in the central nervous system (e.g., an astroglial cell), pigment cells (e.g., a retinal pigment epithelial cell), and progenitor cells thereof (a tissue progenitor cell). There is no particular limitation on the degree of cell differentiation, the age of an animal from which cells are collected, and the like; even undifferentiated progenitors (including somatic stem cells) or even finally differentiated mature cells can be similarly used as a source of the somatic cells according to the present invention. Here, examples of undifferentiated progenitor cells include tissue stem cells (somatic stem cells), such as a neural stem cell, a hematopoietic stem cell, a mesenchymal stem cell, and a dental pulp stem cell.

When iPS cells and/or cells induced to differentiate from them are used as cell materials for transplantation, it is desirable to use somatic cells having the same or substantially the same HLA genotype as that of an individual to be transplanted from the viewpoint of not causing rejection. As used herein, "substantially the same HLA type" means that the HLA genotype is matched to the extent that the transplanted cells can be engrafted when the cells are transplanted. For example, the term includes cases in which the main HLAs (for example, 3 loci of HLA-A, HLA-B and HLA-DR, or 4 loci of them and HLA-C) are the same.

EG cells are defined as pluripotent stem cells derived from spermatogonia (references: Nature, 2008, 456, 344-49). The EG cells can be established by culturing the primordial germ cells in the presence of substances such as LIF, bFGF, a stem cell factor, and the like (Y. Matsui et al. (1992), Cell, 70: 841- 847; J. L. Resnick et al. (1992), Nature, 359: 550-551).

Nt ES cells are cloned embryo-derived ES cells prepared by a nuclear transplantation technique, and have almost the same characteristics as those of fertilized egg-derived ES cells (T. Wakayama et al. (2001), Science, 292: 740-743; S. Wakayama et al. (2005), Biol. Reprod., 72: 932-936; J. Byrne et al. (2007), Nature, 450: 497-502). Specifically, the ES cell established from the inner cell mass of a blastocyst derived from a cloned embryo obtained by replacing the nucleus of a somatic cell with the nucleus of an unfertilized egg is an nt ES (nuclear transfer ES) cell. For the preparation of an nt ES cell, a combination of a nuclear transplantation technique (J.B. Cibelli et al. (1998), Nature Biotechnol., 16: 642-646) and a ES cell production technique (supra) is used (Seika Wakayama et al., (2008), Experimental Medicine, Vol. 26, No. 5, Special edition, pp. 47-52). For nuclear transplantation, reprogramming can be carried out by injecting the nucleus of a somatic cell into an enucleated unfertilized egg of a mammal and culturing the egg for several hours.

Sperm stem cells are testis-derived pluripotent stem cells, which are the origin cells for spermatogenesis. Similar to the ES cells, these cells can be induced to differentiate into various lineages of cells, and have properties such as the potency to produce chimeric mice when grafted into mouse blastocysts (M. Kanatsu-Shinohara et al., (2003) Biol. Reprod., 69: 612-616; K. Shinohara et al., (2004), Cell, 119: 1001-1012). The Sperm stem cell are self-renewable in media containing glial cell line-derived neurotrophic factor (GDNF), and repeated passages can be conducted under the same culture conditions as ES cells so as to provide sperm stem cells (Masanori Takebayashi et al., (2008), Experimental Medicine, Vol. 26, No. 5, Special Edition, pp. 41-46, Yodosha (Tokyo, Japan)).

Muse cells are pluripotent stem cells isolated from mesenchymal cells (Reference: Proc Natl Acad Sci USA. 2010, 107, 8639-43). They can be prepared by the method described in WO2011/007900, and more specifically, they are the pluripotent cells that can be obtained by treating fibroblasts or bone marrow stromal cells with trypsin for a long period of time, preferably 8 hours or 16 hours, and then suspended-culturing them, which cells are positive in SSEA-3 and CD105.

In the present invention, megakaryocytes and platelets induced to differentiate in vitro from "cells capable of differentiating into megakaryocytes" other than the pluripotent stem cells may be used. For example, cells derived from hematopoietic stem cells that can differentiate into megakaryocytes depending on the conditions for inducing differentiation can be used as a starting material. Examples thereof include hematopoietic stem cells, hematopoietic progenitor cells, CD34-positive cells, megakaryocyte/erythroblast progenitor cells (MEP), and megakaryocyte progenitor cells. Cells capable of differentiating into megakaryocytes can be obtained by the well-known methods, including, for example, the isolation from bone marrow, umbilical cord blood, peripheral blood, and the like, as well as the induction to differentiate from the pluripotent stem cells such as ES cells, iPS cells, and the like. When a hematopoietic progenitor cell is used as a cell capable of differentiating into megakaryocytes, the cell can be introduced in advance before the culture step with an oncogene (for example, c-MYC gene) and an apoptosis suppressor gene (for example, BCL-xL gene), an oncogene (for example, c-MYC gene) and a polycomb gene (for example, BMI1 gene), or an oncogene (for example, c-MYC gene), a polycomb gene (for example, BMI1 gene) and an apoptosis suppressor gene (for example, BCL-xL gene) (WO2014/123242, WO2011/034073, WO2012/157586, US2016/002599, US2012/238023, US2014/127815).

The freeze-dried preparation of the present invention is characterized in that the preparation comprises megakaryocytes and platelets. The freeze-dried preparation of the present invention may comprise megakaryocytes and platelets at a desired mixing ratio of them. The mixing ratio of megakaryocytes and platelets is specifically represented by the ratio of the numbers of megakaryocytes and platelets. In the present invention, examples of the ratios of the numbers of megakaryocytes and platelets include, but is not particularly limited to, for example, 1 × 10³ to 1 × 10⁵ megakaryocytes (immortalized megakaryocyte cell lines) to 1 × 10⁶ platelets, and preferably 1 × 10³ to 5 × 10⁴ megakaryocytes (immortalized megakaryocyte cell lines), more preferably 2 × 10³ to 1 × 10⁴, and even more preferably 2 × 10³ to 5 × 10³ megakaryocytes (immortalized megakaryocyte cell lines) to 1 × 10⁶ platelets. More specifically, the freeze-dried preparation of the present invention may comprise 5-6 × 10³ to a maximum of 10⁴ megakaryocytes (immortalized megakaryocyte cell lines) to 2-3 × 10⁶ platelets. In a further embodiment, the ratio of the numbers of megakaryocytes to platelets comprised in the freeze-dried preparation of the present invention is 2 × 10² to 1.5 × 10⁴ platelets, preferably 1.1 × 10³ to 1.5 × 10⁴ platelets to 1 × 10³ megakaryocytes (immortalized megakaryocyte cell lines, mature megakaryocytes, etc.). In a further embodiment, the number of megakaryocytes comprised in the freeze-dried preparation of the present invention may include, but be particularly limited to, for example, 3% or more, preferably 5% or more, more preferably 10% or more of the number of the platelets comprised in the same preparation. The upper limit of the number of the megakaryocytes comprised in the freeze-dried preparation of the present invention may be, but not be particularly limited to, for example, 500% or less, preferably 100% or less, more preferably 50% or less of the number of the platelets comprised in the same preparation.

The mixing ratio of the platelets and the megakaryocytes, which are comprised in the freeze-dried preparation of the present invention, may be adjusted to a desired ratio. In one of the methods for adjusting the desired ratio, the ratio of megakaryocytes to platelets in the preparation may be adjusted so that the number of the megakaryocytes is made larger than that of the platelets or the number of megakaryocytes is made smaller than that of the platelets, depending on the timing of lyophilization during the course of the induction and differentiation from the pluripotent stem cells, for example, the iPS cells. Alternatively, those collected before the release of platelets, that is, at the stage of only megakaryocytes, and those collected by separating and collecting only the released platelets are each prepared, and both may be mixed at a desired ratio (for example, WO2009/122747, US2011/053267). Those skilled in the art can determine whether or not the platelets are released from the megakaryocytes by an optional method, for example, by collecting and observing an aliquot of the culture medium. The released platelets may be also recovered by an optional method. As mentioned, only megakaryocytes or only platelets are recovered, and both are mixed at an arbitrary ratio to obtain a mixed solution, which can be used for the preparation of the present invention. Alternatively, a mixture comprising the megakaryocytes and the platelets in an arbitrary ratio that is prepared by an optional method, for example, by collecting and observing an aliquot of the culture medium, is recovered to obtain the mixture, which may be used for the preparation of the present invention.

The megakaryocytes and the platelets comprised in the freeze-dried preparation of the present invention may be living cells or non-living cells. Further, the megakaryocytes and the platelets comprised in the freeze-dried preparation of the present invention may be disrupted due to the activation in the preparation or subsequent lyophilization thereof, but such freeze-dried preparations are also encompassed in the freeze-dried preparations of the present invention comprising the megakaryocytes and platelets.

The freeze-dried preparation of the present invention may be prepared by inducing differentiation of immortalized megakaryocyte cell lines whose differentiation is induced from iPS cells into platelets (Cell Stem Cell 14, 535-548, April 3, 2014/WO2012/157586, US2014/127815). One of the objects of the present invention is to solve the problem that the differences among lots in the components and amounts of growth factors and the like in PRPs and freeze-dried PRPs derived from an autologous plasma are large, and the effects of the lots are not kept constant. In general, it is important to construct a mass supply system for target cells for the purpose of the spread of regenerative medicine. According to the present invention, the active ingredients are prepared from stem cells in order to guarantee the effect with a certain degree of certainty, and the iPS cells and the immortalized megakaryocyte cell lines may be stocked for a mass supply system, thereby making the business possible in which the stocked materials are induced to differentiate into platelets. In addition, there is a possibility that a third party may obtain the immortalized megakaryocyte cell lines in stock and differentiate them into platelets. Thus, in the preparation method of the present invention, not only stem cells (e.g., pluripotent stem cells (iPS cells, etc.), hematopoietic stem cells), but also immortalized megakaryocyte cell lines induced to differentiate from stem cells in vitro may be used as a starting material, to induce the differentiation into platelets. In this context, the immortalized megakaryocyte cell lines induced to differentiate from stem cells (e.g., pluripotent stem cells (iPS cells, etc.), hematopoietic stem cells) in vitro are used as a starting material, and the immortalized megakaryocyte cell lines are further induced the differentiation in vitro, thereby providing megakaryocytes and platelets, which are also encompassed in "megakaryocytes and platelets induced to differentiate from stem cells in vitro".

The freeze-dried preparation of the present invention may further comprise a growth factor released from the megakaryocytes and/or the platelets. The growth factors according to the present invention include at least one selected from the group consisting of Bone morphogenetic proteins such as BMP2, BMP4, BMP7; Platelet-Derived Growth Factors (PDGFs) such as PDGF-BB; Platelet-Derived Angiogenesis Factors (PDAFs); Transforming Growth Factors (TGFs) such as TGF-β1, TGF-β2, TGF-β3; Vascular Endothelial Growth Factor (VEGF); Epidermal Growth Factor (EGF); Fibroblast Growth Factors (FGFs) such as Basic FGF; Hepatocyte Growth Factor (HGF); Platelet-Derived Endothelial Cell Growth Factor (PD-ECGF); Insulin-like Growth Factor (IGF); and Platelet Factor IV (PF4). In a preferred embodiment, the freeze-dried preparation of the present invention comprises at least BMP2 and/or BMP4. Since these growth factors are released from the megakaryocytes and/or the platelets by the activation of the megakaryocytes and/or the platelets, or by the disruption of the megakaryocytes and/or the platelets due to the lyophilization, the freeze-dried preparation of the present invention may constitute a mixture of the megakaryocytes and the platelets with these growth factors. In a preferred embodiment, the freeze-dried preparation of the invention comprises BMP2 and/or BMP4, and may further comprise, in addition to those, one or more factors selected from other growth factors such as PDGF, PDAF, TGF, VEGF, EGF, FGF, HGF, PD-ECGF, IGF and PF4, thereby exerting a better effect.

Preferred freeze-dried preparations of the present invention include a preparation comprising a platelet/megakaryocyte mixture having BMP-2 in a high content and a platelet/megakaryocyte mixture having BMP-4 in a high content at a desired ratio.

The preparation of the present invention comprises a plural of these growth factors as an active ingredient. Since these growth factors are stable, they can be used even if dissolved or suspended in water, saline or the like and stored at a low temperature, and can be used even if freeze-dried, of which the case, the freeze-dried one is dissolved or suspended in water or saline before use. It would be noted that the growth factors released from activated megakaryocytes and/or platelets are more important as active ingredients, and therefore the preparation in which the megakaryocytes and/or platelets remain, or the preparation in which the platelets are filtered off can also be administered to the subject.

The freeze-dried preparation of the present invention may comprise a stabilizer, a pH adjuster, a tonicity agent, an excipient, and the like, which are well known to those skilled in the art as the generally used agents in freeze-dried preparations, or a plasma. Examples of the stabilizer used in the freeze-dried preparations include sugars such as sucrose, trehalose, sugar alcohols such as sorbitol, amino acids such as L-arginine, water-soluble polymers such as HES and PVP, and nonionic surfactants such as polysorbate. Examples of the pH adjuster include a sodium phosphate buffer and a histidine buffer, examples of the tonicity agent include sodium chloride, and examples of the excipient include mannitol and glycine.

The freeze-dried preparation of the present invention is characterized in that the preparation comprises megakaryocytes and platelets induced to differentiate in vitro from stem cells, and has a tissue repair potency similar to platelet-rich plasma (PRP). Therefore, the freeze-dried preparation of the present invention is expected to be widely effective in the fields of dermatology, plastic surgery, dentistry, and orthopedics, and may be used, for example, to promote the regeneration of intractable cutaneous ulcers, decubitus (bedsores), burns, and necrosis in diabetic patients in dermatology, and dental alveolar bone and gingival, as well as to promote bone adhesion. In addition, the freeze-dried preparation of the present invention may be applied to the damaged parts of cartilage and muscle, to the affected parts during surgery, to the affected parts of osteoarthritis, rheumatism, meniscus tear, and lateral epicondylitis of humerus, and may be administered to improve wrinkles on the skin of face, neck and the like, and to promote hair growth.

### <Production method of freeze-dried preparations>

As a further aspect, the present invention relates to a method for producing a freeze-dried preparation comprising megakaryocytes and platelets, which is characterized in:
(A) preparing a cell mixture of platelets and megakaryocytes, specifically a cell mixture of platelets and immortalized megakaryocytes induced to differentiate from stem cells in vitro, and preferably activating the obtained cell mixture, and
(B) freeze-drying the cell mixture of platelets and megakaryocytes.

The platelets as used in step (A) are platelets induced to differentiate from stem cells in vitro. Platelets induced to differentiate from megakaryocytes induced to differentiate from stem cells in vitro are preferable. Megakaryocytes can be induced to differentiate from stem cells, preferably hematopoietic stem cells or pluripotent stem cells such as iPS cells according to the well-known methods (WO2012/157586, US2014/127815, Nakamura, Eto, et al. Cell Stem Cell 14, 535-548, April 3, 2014). Since the culture provided after differentiating and culturing platelets from megakaryocytes usually comprises platelets and megakaryocytes, this can be used as a cell mixture of platelets and megakaryocytes. Then, the cells and the culture supernatant are separated by centrifugation or the like, and then the selected cell portions are dispersed by pipetting or the like, followed by preferably activating the cells. All of these are preferably performed aseptically.

When activating the cell mixture (platelets and megakaryocytes) in step (A), the stimulation method includes, but is not limited to, the use of thrombin, CaCl₂, TXA₂, epinephrine, and ADP. Preferably, CaCl₂, or a combination of thrombin and CaCl₂ is used. This facilitate to release growth factors comprised in platelets and/or megakaryocytes extracellularly.

In step (B), freeze-drying is performed using a freeze-dryer well known to those skilled in the art. All methods for distilling water can be performed as freeze-drying, which is not particularly limited to any lyophilization. Therefore, the freeze-dried preparation of the present invention includes a preparation in which a moisture portion is removed by an arbitrary method, which comprises no freeze-drying step.

The method for producing the freeze-dried preparation of the present invention is described in detail below. In one example, the method comprises the two steps of (a) activation of a cell mixture of platelets and immortalized megakaryocytes, and (b) freeze-drying treatment of the cell mixture of platelets and immortalized megakaryocytes:
(a) Activation of a cell mixture of platelets and immortalized megakaryocytes
   A cell mixture of platelets into which immortalized megakaryocytes at an order of 10⁵ cells/ml are cultured to differentiate for 6 to 7 days, and the immortalized megakaryocytes is centrifuged at 900 rpm (170G) for 15 minutes, and then the culture supernatant is discarded. Next, the sterilized 0.1% CaCl₂ in Tyrode buffer in an amount of 1/10 of the culture supernatant is added to the centrifugation residue so that the cell concentration is 10 times that at the time of culturing. By pipetting this liquid, the cell mixture of the platelets and the immortalized megakaryocytes is gently loosened, and reacted at 37°C for 1 hour in the incubator as it is, so as to aseptically activate the platelets and the megakaryocytes.
(b) Freeze-drying treatment of the cell mixture of platelets and immortalized megakaryocytes

The cell mixture of the activated platelets and immortalized megakaryocytes is transferred to a sterile culture vessel equipped with a membrane filter, and this is used as a sample for the freeze-drying treatment. First, the sample for the freeze-drying treatment is sufficiently pre-frozen at -60°C that is below the eutectic point over a day and night. Next, the sample is subjected to a freeze-dryer (EYELA FDU-1200) set to -45°C or lower in advance, and is freeze-dried over one day and night under a reduced pressure by a vacuum pump to produce a preparation.

The freeze-dried preparation of the present invention may comprise a stabilizer, a pH adjuster, a tonicity agent, an excipient, and the like, which are well known to those skilled in the art as the generally used agents in freeze-dried preparations, or a plasma. Examples of the stabilizer used in the freeze-dried preparations include sugars such as sucrose, trehalose, sugar alcohols such as sorbitol, amino acids such as L-arginine, water-soluble polymers such as HES and PVP, and nonionic surfactants such as polysorbate. Examples of the pH adjuster include a sodium phosphate buffer and a histidine buffer, examples of the tonicity agent include sodium chloride, and examples of the excipient include mannitol and glycine.

In another example for producing the freeze-dried preparation of the present invention, the pluripotent stem cells are induced to differentiate into pluripotent hematopoietic progenitor cells by a method known per se according to the present invention, and then immortalized megakaryocyte cell lines prepared by the cell introduction of the above-mentioned series of genes (c-MYC/BMI1/BCL-xL, and the like) with, for example, a vector whose expression can be controlled in a doxycycline-dependent manner are cultured and proliferated in the presence of doxycycline (i.e., in the condition in which c-MYC/BMI1/BCL-xL and the like are expressed), followed by culturing the proliferated cells in a medium without doxycycline (i.e., in the condition in which the expressions of c-MYC/BMI1/BCL-xL and the like are suppressed) so as to induce the differentiation of the immortalized megakaryocyte cell lines into platelets (medium conditions: for example, WO2012/157586, US2014/127815, Nakamura, Eto, et al. Cell Stem Cell 14, 535-548, April 3, 2014, WO 2019/009364). The immortalized megakaryocyte cell lines still remain in the culture under these conditions (As an example, immortalized megakaryocyte cell lines at 2-3 × 10⁵/ml are inoculated and subjected to doxycycline-free medium conditions for production of platelets. Platelets at about 2-3 × 10⁶/ml are produced in the flask. 10-15 platelets are produced per immortalized megakaryocyte cell line. An immortalized megakaryocyte cell line at about 5-6 × 10³ to 10⁴/ml remains per flask. For example, the cell culture (a mixture of the platelets and the immortalized megakaryocyte cell lines) is recovered after 4 day or the following days (e.g., 4th, 5th, 6th, 7th day) after the induction for differentiation (removal of doxycycline). The resulting mixture of the platelets and the immortalized megakaryocytes may be activated (by e.g., CaCl₂ 1mM + thrombin 1U), to provide a mixture comprising activated megakaryocytes and platelets.

As described above, the freeze-dried preparation of the present invention may further comprise growth factors released from megakaryocytes and/or platelets in addition to megakaryocytes and platelets to constitute a mixture. These growth factors are released from megakaryocytes and/or platelets by the activation of the megakaryocytes and/or platelets, or by the disruption of the megakaryocytes and/or platelets due to lyophilization. The mixture according to the present invention can be prepared by stimulating megakaryocytes and/or platelets derived from pluripotent stem cells to release growth factors. Techniques for stimulation include thrombin, CaCl₂, TXA₂, epinephrine, and ADP.

Preferred methods for producing the freeze-dried preparation of the present invention include a production method that comprises mixing a platelet/megakaryocyte mixture having a high BMP-2 content and a platelet/megakaryocyte mixture having a high BMP-4 content in a desired ratio.

As yet another example for producing the freeze-dried preparation of the present invention, the following can be mentioned.
A. The megakaryocytes and/or platelets as used in the present invention may be prepared by selecting ES cell clones or iPS cell clones having a high ability to produce the sac-like structure enclosing hematopoietic progenitor cells obtainable by inoculating human-derived ES cells or iPS cells onto feeder cells such as C3H10T1/2 cells or OP9 cells, and culturing those cells under conditions suitable for inducing the differentiation of hematopoietic progenitor cells, for example, for 14 to 17 days in the presence of VEGF; separating cells forming the septum of the sac-like structure from hematopoietic progenitor cells, both of which are produced by the ES cell or iPS cell clones; inoculating the obtained hematopoietic progenitor cells onto feeder cells; and culturing the hematopoietic progenitor cells under conditions suitable for inducing the differentiation of blood cells (WO2009/122747). In addition, megakaryocytes and/or platelets may be prepared by subjecting human-derived ES cells or iPS cells to a liquid culture to form hematopoietic progenitor cells in the embryoids thereof; and further culturing the embryoids, for example, for 5 to 7 days in the presence of TPO and SCF (ibid.).
B. The mature megakaryocyte cells and/or platelets as used in the present invention may be prepared by; introducing a foreign oncogene such as a MYC family gene, or an oncogene and a polycomb gene such as BMI1, and forcing expression of the oncogene, or the oncogene and the polycomb gene;
   so as to amplify cells induced to differentiate from human ES cells or iPS cells, for example, pre-multinuclear megakaryocyte progenitor cells (WO2011/034073, WO2011/034073, JP-A-2015-130866).
C. The polyploidized megakaryocytes as used in the present invention may be prepared by;
   forcing expression of an apoptosis suppressor gene such as a BCL-XL gene in the megakaryocyte before polyploidization; and
   culturing the cells with subjecting to (a) treatment with an actomyosin complex function inhibitor such as blebbistatin, (b) treatment with an ROCK inhibitor such as Y27632, (c) treatment with an antagonist to an aromatic hydrocarbon receptor (an aryl hydrocarbon receptor) such as StemRegenin (SR)-1-(4-(2-(2-(benzo[b]thiophen-3-yl)-9-isopropyl)-9H-purin-6-ylamino)ethyl)phenol ), or (d) treatment with an HDAC inhibitor such as valproic acid (WO2012/157586, JP-A-2019-026591). The platelets as used in the present invention may be produced by inhibiting the forced expression of the apoptosis suppressor gene in the polyploidized megakaryocytes obtained above, or removing the apoptosis suppressor gene from the cells, and then culturing those cells in a medium supplementing with a ROCK inhibitor and/or an actomyosin complex function inhibitor (ibid.).
D. The platelets as used in the present invention may be produced from the megakaryocytes by (a) maintaining the megakaryocytes in a holding portion having a porous structure through which a culture medium can pass: and (b) culturing the megakaryocytes while passing the culture medium through the holding portion from the anterior chamber provided on one side of the holding portion to the flow path portion provided on the other side of the holding portion JP-A-2013-031428).
E. The megakaryocytes as used in the present invention may be produced by culturing pluripotent stem cells on C3H10T1/2 cells in a culture medium comprising VEGF to induce the differentiation into hematopoietic progenitor cells, and culturing the hematopoietic progenitor cells while forcing the expression of the apoptosis suppressor gene and the oncogene in the cells, followed by culturing the obtained cells while stopping the forced expression of the apoptosis suppressor gene and the oncogene (WO2014/123242, US2016/002599). The platelets as used in the present invention can be produced by recovering from the culture of the megakaryocytes obtained as shown above (ibid.).
F. The megakaryocytes as used in the present invention may be produced by culturing, for example, immature megakaryocytes produced from hematopoietic progenitor cells derived from pluripotent stem cells in a culture medium comprising an inhibitor of the C family of ABC transporters, for example, an ABC transporter inhibitor such as probenecid (JP-A (Saihyo) 2014/168255).
G. The megakaryocytes as used in the present invention may produced by culturing a cell population including megakaryocytes or cells capable of differentiating into megakaryocytes under conditions in which cells that do not express genes specifically expressed in megakaryocytes are lethal, thereby preparing a culture comprising megakaryocytes or megakaryocyte progenitor cells (WO2016/143836, US 2018/044634). The platelets as used in the present invention can be produced by using the megakaryocytes obtained as shown above (ibid.).
H. The platelets as used in the present invention may be produced by contacting the megakaryocyte cells or the progenitor cells thereof with a high platelet production promoter comprising one or more aromatic hydrocarbon receptor (aryl hydrocarbon receptor; AhR) antagonists and one or more ROCK (Rho binding kinase) inhibitors (WO2016/204256).

The megakaryocytes and/or platelets as used in the present invention may be produced by culturing progenitor cells of the megakaryocytes or platelets in the presence of IL-1α, and collecting the generated megakaryocytes or platelets (JP-A-2017-122049).

I. The platelets as used in the present invention may be produced by a culture method comprising a step of contacting megakaryocytes and/or progenitor cells thereof with a platelet production promoter comprising one or more substances selected from the group consisting of a Wnt inhibitor and an FLT inhibitor (WO2017/131230).

J. The platelets as used in the present invention may be produced by a culture method comprising a step of contacting megakaryocytes and/or precursor cells thereof with a platelet production promoter comprising one or more AhR antagonists selected from the group consisting of a receptor-type tyrosine kinase inhibitor, a vanilloid receptor inhibitor, a PDGFR inhibitor, a TrioN inhibitor, a SIRT2 inhibitor, an H+,K+-ATPase inhibitor, a benzodiazepine inverse agonist, and a nerve development promoter (JP-A-2019-026591).

K. The platelets as used in the present invention may be produced by a method for producing platelets comprising a step of culturing megakaryocytes in a platelet production medium, wherein the culturing step comprises stirring the platelet production medium in a vessel by using a stirring blade, wherein the stirring step comprises reciprocating the stirring blade to satisfy one or more indexes selected from: (a) a turbulent flow energy from about 0.0005 m²/s² to about 0.02 m²/s²; (b) a shear stress from about 0.2 Pa to about 6.0 Pa; and (c) a Kolmogorov scale of about 100 µm to about 600 µm (WO2019/009364).).

### <Pharmaceutical composition>

As yet another aspect, the present invention relates to a pharmaceutical composition which comprises the freeze-dried preparation of the present invention.

The freeze-dried preparation of the present invention has a tissue repair potency similar to that of the platelet-rich plasma (PRP) prepared from blood and the freeze-dried preparation thereof. Therefore, the pharmaceutical composition of the present invention is expected to be widely effective in the fields of dermatology, plastic surgery, dentistry, and orthopedics, and may be used, for example, to promote the regeneration of intractable cutaneous ulcers, decubitus (bedsores), burns, necrosis in diabetic patients in dermatology, and dental alveolar bone and gingival, as well as to promote bone adhesion. In addition, the pharmaceutical composition of the present invention can be applied to the damaged parts of cartilage or muscle, to the affected parts during surgery, to the affected parts of osteoarthritis, rheumatism, meniscus tear, and lateral epicondylitis of the humerus, and may be administered to improve wrinkles on the skin of face, neck and the like, and to promote hair growth. Since the freeze-dried preparation of the present invention comprises BMP2 and/or BMP4 in an abundant amount, the composition is more suitably used to promote the bone adhesion, or to treat or prevent the skin diseases among the above-mentioned uses. The pharmaceutical composition of the present invention preferably comprises the freeze-dried preparation of the present invention in an amount effective for promoting the bone adhesion, or treating or preventing the skin disease.

Fracture is to break a bone, and also includes a cracked bone, a bone defect, and a depressed bone. Since the bone and its surroundings are rich in nerves and blood vessels, as a symptom of the fracture, pain and swelling usually appear at the site of the fracture, and the site becomes immobile or the appearance is deformed in the severe fracture. The fractures include an open fracture in which the skin is torn at the same time as the fracture and the fractured part is exposed, a crushed fracture in which the fractured part is complicatedly crushed, and an incomplete fracture in which there are only cracks without dislocation (gap). The fracture causes damages on the bone, the periosteum that covers the bone, or the surrounding soft tissue, and bleedings. The bleeding blood contains a kind of fibers, which induce differentiation of osteoblasts that produce and secrete collagens and proteoglycans, and form collagen fibers that serve as pillars of the resulting bone tissues to be constructed. The process of reconstructing the bone tissues is "bone adhesion". Bone adhesion is synonymous with bone fusion.

In the present invention, "promoting a bone adhesion" means a process of reconstructing a bone fragment destroyed and separated by a fracture, restoring the continuity of the broken bone tissue, and regaining the support and strength of the bone. Further, in the present invention, "promoting a bone adhesion" means, in addition to this mere process of regaining the support and strength of bone, a process of reconstructing a bone fragment destroyed and separated by a fracture, restoring the continuity of the broken bone tissue, and regaining the support and strength of the bone earlier and more appropriately than the natural reconstruction of the bone tissue as mentioned above.

In general, a fracture section is healed after predetermined periods of the following phases 1 to 5: Fracture hematoma phase wherein periosteum, bone marrow, muscles and blood vessels are damaged and then hematoceles are formed (1st stage, 8 to 10 days), Initial callus formation phase wherein proliferated tissues are clearly distinguishable from the surroundings, resulting in the hardness of fibrocartilages (2nd stage, 10 to 25 days), Periosteal cell proliferation phase wherein the initial callus shrinks and bones are formed as a whole (3rd stage, 20 to 60 days), Hardening phase wherein the spongy bone-like callus changed into hard bones (4th stage, 50 days to 6 months), and Modification phase wherein the complete bones wrapping with normal periosteum are formed (5th stage, 4 to 12 months).

In the present invention, the term "earlier and more appropriately than the natural reconstruction of the bone tissue" according to "promoting a bone adhesion" means an embodiment wherein the bone adhesion is induced earlier and more reliably than those healing processes.

In the present invention, "skin disease" means a wound on the skin, such as an intractable cutaneous ulcer, a decubitus (bedsore), a burn, and a necrosis or gangrene in diabetic patients. When the pharmaceutical composition of the present invention is administered intradermally, a plural of proteins having a physiological activity such as growth factors are released to increase in capillary lymph vessels in the skin and to cause skin fibroblasts to migrate, thereby regenerating capillary lymphatic vessels, and capillary blood vessels and new cutaneous granule tissues, and therefore the composition is expected to treat or prevent skin diseases such as skin ulcers, skin decubitus, and skin damages that occur during lower limb ischemia. Furthermore, the composition can be administered to a subject to improve wrinkles on the skin of face, neck and the like, and to promote hair growth.

To date, there are no reports of producing a wound healing preparation similar to blood donation PRPs from platelets induced to differentiate from stem cells such as iPS cells, and examining its efficacy and safety. Through the development of donor-independent blood platelet preparations for transfusion (Ito Y, Nakamura S, Sugimoto N. et al, Cell, 174 (3): 636-648, 2018: https://www. ncbi.nlm.nih.gov/pubmed/30017246), the present inventors have attained the stage wherein a clinical research is begun on iPS artificial autologous platelet transfusion therapy for patients with aplastic anemia for which no donor can be found using autologous iPS cells as a starting material.

In addition, platelets, which do not have any cell nuclei, are positioned as functional cells that do not proliferate scientifically, or as cell fragmentation preparations in the pharmaceutical affairs, and are indicated to be used after irradiation with a radiation of 15-25 Gy or more in transfusion preparations for the purpose of killing contaminated nucleated cells. Therefore, the invention is considered to have an extremely large advantage since it is highly safe in transplantation (graft) medicine and regenerative medicine using iPS cells (reduction of cancer risk, etc.).

The megakaryocytes and platelets which are the active ingredients of the pharmaceutical composition of the present invention can be prepared in the same manner as the freeze-dried preparation comprising megakaryocytes and platelets which are induced to differentiate in vitro from stem cells. The definitions of the stem cells and the like and the method for producing them are similar to those in the freeze-dried preparation of the present invention. The "amount effective for promoting a bone adhesion" of megakaryocytes and platelets in the pharmaceutical composition of the present invention may be any amount as long as it can promote the bone adhesion, and the numerical values are not particular. The same is true to the "amount effective for treating or preventing a skin disease". For example, the megakaryocytes and platelets may be administered at one dose of about 2 × 10⁶ to about 2 × 10¹⁰ cells, preferably about 2 × 10⁷ to about 2 × 10⁹ cells of the platelets. In a further embodiment, for the treatment of skin wounds, the megakaryocytes and platelets may be administered at one dose of about 2 × 10⁶ to about 2 × 10¹⁰ cells, preferably about 2 × 10⁷ to about 2 × 10⁹ cells (e.g., 4.5 × 10⁷ cells) of platelets per 1 cm² of wound. In another embodiment, the number of the megakaryocytes administered per dose may include, but be not particularly limited to, for an example, 3% or more, preferably 5% or more, more preferably 10% or more than the number of the platelets administered per dose. The upper limit of the number of the megakaryocytes administered per dose may include, but be not particularly limited to, for example, 500% or less, preferably 100% or less, more preferably 50% or less of the number of the platelets administered per dose. The dose administered depends on a variety of factors, including age, body weight, gender of the subject, disease/condition to be treated, and the extent and severity thereof.

The pharmaceutical composition of the present invention can further comprise growth factors released from the megakaryocytes and/or platelets in addition to the megakaryocytes and platelets. The growth factors in the present invention include at least one selected from the group consisting of Bone morphogenetic proteins such as BMP2, BMP4, BMP7; Platelet-Derived Growth Factors (PDGFs) such as PDGF-BB; Platelet-Derived Angiogenesis Factors (PDAFs); Transforming Growth Factors (TGFs) such as TGF-β1, TGF-β2, TGF-β3; Vascular Endothelial Growth Factor (VEGF); Epithelial Cell Growth Epidermal Growth Factor (EGF); Fibroblast Growth Factors (FGFs) such as Basic FGF; Hepatocyte Growth Factor (HGF); Platelet-Derived Endothelial Cell Growth Factor (PD-ECGF); Insulin-like Growth Factor (IGF); and Platelet Factor IV (PF4). In a preferred embodiment, the pharmaceutical composition of the invention comprises at least BMP2 and/or BMP4. The definitions of these growth factors and the method for producing them are similar to those in the freeze-dried preparation of the present invention.

The compositions of the invention may be formulated with a pharmaceutically acceptable carrier. For example, the pharmaceutical composition of the present invention may be administered alone or as a pharmaceutical formulation component. The test compound may be formulated for administration in any convenient manner for use in medicaments. As suitable medicaments, one or more pharmaceutically acceptable sterile isotonic or non-aqueous solutions (e.g., Balanced Salt Solution (BSS)), dispersions, suspensions or emulsions may be combined, or sterile powders that may be returned to the dispersions or sterile injectable solutions that may comprise oxidants, buffers, bacteriostatic agents, solutes or suspensions or thickeners, may be combined just before use.

The composition of the present invention may be a formulation suitable for therapeutic use, such as a formulation without or substantially without any pyogenic substance, and a sterile formulation. Upon administration, the medicament as used in the present invention may be in a sterile and physiologically acceptable form without any pyogenic substance. The pharmaceutical compositions of the invention may be administered in the forms of suspensions, gels, colloids, slurries, or the mixtures.

The base materials as used for the pharmaceutical composition and the freeze-dried preparation of the present invention are as follows.

The invention can typically comprise the step of administering the pharmaceutical composition or freeze-dried formulation of the invention as a graft to the affected area of the fracture. In certain embodiments, the invention comprises an active ingredient dispersed in a biodegradable polymer base. Examples of the biodegradable polymer may include, for example, a poly (lactic acid-co-glycolic acid) (PLGA) copolymer, a biodegradable poly (DL-lactic acid-co-glycolic acid) film, or a PLLA/PLGA polymer substrate. The ratio of glycolic acid monomers in the polymer is about 75/25, 60/40, 50/50, 40/60, 25/75, and more preferably about 50/50 weight percent. The PLGA copolymer may be from about 20, 30, 40, 50, 60, 70, 80 to about 90% by weight of the biodegradable graft. The PLGA copolymer may be from about 30 to about 50% by weight, preferably about 40% by weight of the biodegradable graft. The pharmaceutical compositions and freeze-dried formulations of the invention may be grafted in conjunction with a biocompatible polymer such as polylactic acid, poly (lactic acid-co-glycolic acid), 50:50 PDLGA, 85:15 PDLGA, and INION GTR ^{®} biodegradable membranes (biocompatible polymer mixture). See also Lu, et al. (1998) J Biomater Sci Polym Ed 9: 1187-205; and Tomita, et al. (2005) Stem Cells 23: 1579-88.

As another aspect, the present invention relates to a method for promoting a bone adhesion, or treating or preventing a skin disease, which comprises administering the freeze-dried preparation comprising megakaryocytes and platelets of the present invention to a subject in need of such treatment, and preferably a method which comprises administering to such a subject the freeze-dried preparation of the present invention in an effective amount.

Further, as another aspect, the present invention relates to the freeze-dried preparation comprising megakaryocytes and platelets of the present invention for promoting a bone adhesion, or treating or preventing a skin disease.

In yet another aspect, the present invention relates to use of the freeze-dried preparation comprising megakaryocytes and platelets of the present invention for manufacturing a medicament for promoting a bone adhesion, or treating or preventing a skin disease.

As used herein, the term "subject" or "patient" means humans and non-human animals, and examples of non-human animals include, but not be limited to, pet animals such as primates, dogs, cats, birds, and the like, mice, rats, Guinea pigs, rabbits, chickens, pigs, horses, sheep and cattle. Preferably, the subject or patient is a human and a pet animal, and more preferably a human.

All references cited herein including the publications, the patent documents, and the like are incorporated herein by reference in their entireties comparably as if those references were specifically and individually indicated to be incorporated by reference.

Hereinafter, the present invention is illustrated in detail by means of the working examples, but it should be noted that those examples would not limit the scope of the present invention, and merely illustrate the invention.

### Examples

### Reference example 1

### Establishment of immortalized megakaryocytes and induction of differentiation into platelets

The differentiation into platelets were induced from the immortalized megakaryocyte cell lines established in Cell Stem Cell. 2014 Apr 3; 14 (4): 535-48. Specifically, the iPS cell line Sev2 prepared by introducing Yamanaka 4 factors into the purchased fetal fibroblasts with Sendai virus were cultured on CH3T10T1/2 cells for 11 to 14 days in the presence of VEGF according to the report (Takayama et al., Blood 111, 5298-5306 (2008); Takayama et al., J. Exp Med 207, 2817-30, 2010), to obtain the pluripotent hematopoietic progenitor cells by Sac method. The pluripotent hematopoietic progenitor cells were infected with c-MYC and BMI-1 using the lentiviral vector-derived viruses (2 types). The BCL-xL gene was also introduced into the proliferating cell population to obtain the cell population that proliferated while maintaining the CD41a/CD42b expressions for 1 month or longer, which is called immortalized megakaryocyte cell lines (imMKCLs). ImMKCL Clone 7 was used for the following tests. ImMKCL Clone 7 was grown by culturing them in the presence of doxycycline (i.e., in the condition in which c-MYC/BMI-1/BCL-xL were expressed), and then the clone was cultured for 6 to 7 days under the medium conditions without doxycycline (i.e., in the condition in which the expressions of c-MYC/BMI-1/BCL-xL were suppressed), so as to induce the differentiation into platelets. The culture conditions were according to Cell Stem Cell. 2014 Apr 3; 14 (4): 535-48.

### Example 1

### Production of freeze-dried preparation comprising megakaryocytes and platelets

### Example 1-1: Activation of cell mixture of platelets and immortalized megakaryocytes

The cell mixture (120 ml) of the platelets and the immortalized megakaryocytes obtained by the culture for differentiation in the absence of doxycycline as described above (Ito Y, Nakamura S, et al., Cell 174 (3): 636-648, 2018) from immortalized megakaryocyte cell line Clone 7 (Also mentioned as Sev2 clone: Nakamura et al., Cell Stem Cell. 2014 Apr 3; 14 (4): 535-48; Ito Y, Nakamura S, et al., Cell 174 (3): 636-648, 2018) at 2.0 × 10⁵ cells/ml (25 ml × 5, 125 ml triangular flask) 6 to 7 days after the liquid culture was centrifuged at 900 rpm (170G) for 15 minutes, and then the culture supernatant was discarded. Next, the sterilized 0.1% CaCl₂ in Tyrode buffer (12 ml) was added to the centrifugation residue in an amount of 1/10 of the culture supernatant so that the cell concentration was 10 times that at the time of culturing. The centrifugation residue was loosened by pipetting to obtain the cell mixture of platelets and immortalized megakaryocytes, and then reacted at 37°C for 1 hour in the incubator as it is, so as to aseptically activate the platelets and megakaryocytes. As a result, the cell mixture (sterile) of the platelets and the immortalized megakaryocytes was obtained.

### Example 1-2: Freeze-drying process of a cell mixture of platelets and immortalized megakaryocytes

The cell mixture of the platelets and the immortalized megakaryocytes (sterile) prepared in Example 1-1 was dispensed into the lyophilization tubes (1 ml × 9, and 750 µl × 4), and the tubes were transferred to the sterile culture vessel equipped with the membrane filter to provide the samples for freeze-drying process. These samples for freeze-drying process were first sufficiently pre-frozen at -60°C below the eutectic point over a day and night or more. Next, the products were subjected to the freeze-dryer (EYELA FDU-1200) previously set at not more than -45° C, and freeze-dried over one day and night under the reduced pressure by the vacuum pump to prepare the desired freeze-dried preparations.

### Example 2

### Confirmation of the bone adhesion effect of the freeze-dried preparations comprising the megakaryocytes and the platelets

The bone adhesion-promoting effect of the freeze-dried preparations comprising the iPS cell-derived immortalized megakaryocytes and platelets prepared in Example 1-2 was confirmed using the rat lumbar fusion model that was prepared according to the method described in Shiga, et al. Sci Rep. 2016 Nov 11; 6: 36715. Specifically, SD rats (8 weeks old) was used to create the lumbar fusion model. The skin incision was made at the level of the 4th-6th lumbar vertebrae spinous process, and the longitudinal fascial incision was made 1.5 mm laterally from the center of the spinous process. The back muscles were bluntly deployed to expose the 4th-6th lumbar transverse processes. 0.5 ml of an artificial bone hydroxyapatite collagen complex: Refit (Hoya Corporation, Tokyo, Japan), was crushed, and the material was mixed with 10 µg of the freeze-dried preparation as mentioned above, followed by transplanting the mixture to both sides of the lumbar spine. The fascia and the skin were sutured to complete the model creation. The bone fusion was evaluated by radiography at 2, 4 and 6 weeks after the operation.

The X-ray image of the rat spine 2 weeks after the graft is shown in Figure 1. When the freeze-dried preparation comprising the iPS cell-derived immortalized megakaryocytes and platelets was grafted between the rat spinal transverse processes, the bone crosslinks were observed between the transverse processes 2 weeks after the graft. This result suggests that the freeze-dried preparation comprising the iPS cell-derived immortalized megakaryocytes and platelets has an excellent bone adhesion-promoting effect and bone-forming potency.

### Example 3

### Measurement of growth factor concentrations in the preparation

In the preparation process of the freeze-dried preparation of Example 1-2, the concentration of the growth factors comprised in the preparation were measured by ELISA immediately after the activation of megakaryocytes/platelets, and after the freeze-drying process. The kits used is shown in Table 2. The concentration of the megakaryocytes at the time of the activation was 1 × 10⁶ cells/ml. The results are shown in Table 3. In Table 3, the concentrations of the growth factors immediately after the activation (before FD) shows the concentrations in 1 ml of the cell mixture. The concentrations of the growth factor after the freeze-drying process (after FD) shows the concentration when each vial is filled up to 1 ml with a purified water. Even after the freeze-drying process, the amount of each cytokine comprised in the preparation was well maintained.

**Table 2**

| ( Kits : R&D SYSTEMS) | | | |
|---|---|---|---|
| ▪ TGF *β* 1 | DY240-05 | for Human TGF *β* 1 | |
| ▪ PDGF-BB | DY220 | for Human PDGF-BB | |
| ▪ EGF | DY236-5 | for Human EGF | |
| ▪ BMP-2 | DY355-05 | for Human BMP-2 | |

**Table 3**

| Cytokines | before FD | | after FD | |
|---|---|---|---|---|
| TGF *β* 1 | 23, 620 | | 25, 252 | |
| PDGF-BB | 2, 812 | | 2, 079 | |
| EGF | 792 | | 642 | |
| BMP-2 | 2, 921 | | 2, 331 | |

| | | | | |
|---|---|---|---|---|
| (pg/ml) | | | | |

### Example 4

### Measurement of BMP2 and BMP4 concentrations

In the preparation process of the freeze-dried preparation of Example 1-2, the concentrations of BMP2 and BMP4 comprised in the preparation were measured immediately after the activation of the megakaryocytes/platelets (before FD). In addition, the PRPs prepared from the human peripheral blood comprising the same number of the platelets (2 individuals: human PRP1 and human PRP2) were activated in a manner similar to Example 1-2, and the concentrations of BMP2 and BMP4 in the PRPs were measured. As a control, the differentiation medium (doxycycline was removed from the growth medium, and SR1 750uM, Y27632 10mM and KP457 15mM were added thereto) was used.

The result is shown in Figure 2. The supernatants of the activated immortalized megakaryocytes and platelets derived from iPS cells were higher in the BMP2 and BMP4 concentrations than those in the activated PRPs derived from human peripheral blood comprising the same number of the platelet. Individual differences were recognized in the BMP2 and BMP4 concentrations in the activated PRPs derived from human peripheral blood.

### Example 5

### Detection of cytokines released from iPS cell-derived mature megakaryocytes and platelets

The shaking culture was performed on the iPS cell-derived immortalized megakaryocyte cell line imMKCL prepared in Reference Example 1 in the differentiation medium (doxycycline was removed from the growth medium, and SR1 750uM, Y27632 10mM and KP457 15mM were added thereto) to provide the mature megakaryocytes and platelets (MMK-PLT). We investigated whether or not the MMK-PLT released the wound healing-promoting cytokines similarly to the human peripheral blood-derived PRPs. Specifically, the mRNAs were first recovered from the MMK-PLT differentiated on the 0th, 4th, and 6th days, and the gene expressions of TGF-β1, PDGF-BB, and EGF were analyzed over time using the RT-qPCR method. Then, CaCl₂ and thrombin were added to each MMK-PLT to activate them, and the supernatant was collected by the centrifugation. The concentrations of TGFβ, PDGF-BB, EGF, and bFGF proteins comprised in this supernatant were measured by the ELISA method.

The results obtained are shown in Figures 3 and 4.

The gene expression of each cytokine was markedly increased until the 6th day in accordance with the maturation of the megakaryocytes. The protein concentration of each cytokine released by the activation was also increased in accordance with the maturation, but no significant difference was recognized between the 4th and 6th days. These results suggested that the MMK-PLT after the 4th day of the differentiation comprised each cytokine in an abundant amount.

### Example 6

### In vitro wound healing assay

During the proliferative phase of the wound healing, fibroblasts that exist in the wound constitute extracellular matrixes such as a collagen. The effect of the MMK-PLT on the proliferation potency of the human primary culture fibroblasts was analyzed.

Specifically, the supernatant of the activated MMK-PLT (on the 6th day) prepared in Example 5 was added to the culture medium (serum-free) of the human primary culture fibroblasts, and the number of the cells were counted on the 3rd and 5th days. The number was compared with those in the control group wherein no material was administered and the positive control group wherein FBS was administered (n = 3).

The results obtained are shown in Figure 5.

The number of the cells in the group wherein MMK/PLT was administered was increased significantly compared with that in the control group. In addition, when the culture was continued for 5 days, the proliferation potency in the group wherein FBS was administered was reduced, but the proliferation potency in the group wherein MMK/PLT was administered was continued, and a significant difference between the cell numbers in both groups was recognized.

### Example 7

### In vivo wound healing assay

We investigated whether or not the MMK-PLT actually promoted the wound healing using an animal model.

Specifically, the 6 mm-sized full-thickness skin defect was created on the left and right backs of the mice suffering from the immunodeficiency (NOD/SCID IL2R defect) (obtained from Claire Japan). The MMK-PLT (prepared as described in Example 5) was recovered on the 5th day of the differentiation, and the immortalized megakaryocyte cell lines at the beginning of the culture for differentiation at 8 × 10⁵ cell numbers were subcutaneously injected around the skin defective surface. In addition, the immortalized megakaryocyte cell lines at the beginning of the culture for differentiation at 2 × 10⁵ cell numbers were mixed with the basement membrane matrix preparation, and the mixture was applied to the wound surface, followed by dressing the aria with the polyurethane film. The total dose for one wound was 1 × 10⁶ cell numbers of the immortalized megakaryocyte cell lines at the beginning of the culture for differentiation, which correspond to 3 × 10⁶ cell numbers of the immortalized megakaryocyte cell lines at the beginning of the culture for differentiation per 1 cm² wound. From Table 5 in Example 10, the numbers to be administered at the time of administration (on the 5th day of the differentiation) were estimated as about 2.2 × 10⁶ megakaryocytes and about 1.5 × 10⁷ platelets per wound (about 6.6 × 10⁶ megakaryocytes and about 4.5 × 10⁷ platelets per 1 cm2 wound). The silicone rubber ring was sewn around to prevent the shrinkage of the wound. The size of the wound surface was measured every 3 days for 10 days, and the reduction rate was analyzed. The rate was compared with that of the control group wherein PBS was administered (n = 4).

The results obtained are shown in Figure 6.

The group wherein the MMK/PLT was administered tended to have a higher reduction rate of the wound area compared with that in the PBS group, and a significant difference was recognized on the 7th day. This result suggests that MMK/PLT has a possibility to promote any wound healing.

### Example 8

### Production No. 2 of freeze-dried preparation comprising megakaryocytes and platelets

The immortalized megakaryocyte cell line (NC13X) induced to differentiate from the iPS cell line (MKCL21 #), which was different from that of Example 1 was used. 2.3 × 10⁷ NC13X cells were subjected to the differentiation and culture (Ito Y, Nakamura S, et al., Cell 174 (3): 636-648, 2018) in the absence of doxycycline similarly to Example 1. The cell mixture of the platelets and the megakaryocytes on the 6th day of the differentiation was centrifuged, the culture supernatant was discarded, and the sterilized 0.1% CaCl² in Tyrode buffer (23 ml) was added to the resulting mixture of the platelets and the megakaryocytes. The platelets and the megakaryocytes were aseptically activated by incubation at 37°C for 1 hour. The mixture of the activated platelets and megakaryocytes was freeze-dried in a manner similar to Example 1-2 to obtain the freeze-dried preparation.

The obtained freeze-dried preparation was diluted with the distilled water to the concentration of 1.0 × 10⁶ cells/ml as NC13X cells at the beginning of the differentiation and culture, and the concentrations of PDGF, FGF, TGF-β and VEGF were measured by ELISA. The results are shown in Table 4.

**Table 4**

| **Cytokine concentrations (ng/ml)** | |
|---|---|
| **PDGF** | **15.99** |
| **FGF** | **2.12** |
| **TGF-β** | **57.82** |
| **VEGF** | **0.07** |

The results suggested that the obtained freeze-dried preparation comprised the major cytokines in a sufficient amount.

### Example 9

### Confirmation No. 2 of bone adhesion effect of freeze-dried preparation comprising megakaryocytes and platelets

Similar to Example 2, the rat lumbar fusion model was created, and the bone adhesion-promoting effect of the freeze-dried preparation comprising the megakaryocytes and the platelets prepared from the iPS cell-derived immortalized megakaryocyte cell line prepared in Example 8 was evaluated. Specifically, the lumbar transverse processes of the SD rats (n = 4) were exposed, and to the right side and to the left side, only the artificial bone hydroxyapatite collagen complex: Refit (Hoya Corporation, Tokyo, Japan), and the mixture of Refit and the freeze-dried preparation of Example 8 were grafted respectively, followed by measuring and comparing the amounts of the new bone formations on the right and left sides by ImageJ 5 weeks after the grafts. As a result, the amount of the new bone formation was significantly increased on the side grafted with the freeze-dried preparation comprising the megakaryocytes and the platelets derived from the iPS cell-derived immortalized megakaryocyte cell line (Figure 7).

Taking also account of the result of Example 2, the results as mentioned above suggested that the freeze-dried preparations comprising the megakaryocytes and the platelets derived from the iPS cell-derived immortalized megakaryocyte cell lines, regardless of the type of iPS cell line, comprise cytokines that promote tissue-repairs and bone formations in abundant amounts, and have an excellent bone-forming potency.

### Example 10

### Analysis of the mixing ratio of megakaryocytes and platelets

Similar to Example 1, immortalized megakaryocyte cell line Clone 7 was subjected to the culture for differentiation in the absence of doxycycline (Ito Y, Nakamura S, et al., Cell 174 (3): 636-648, 2018)), and the numbers of the mature megakaryocytes (CD41+) and the platelets (CD41+CD42+) in the culture were measured over time with the flow cytometer, thereby calculating the ratio of the platelets to the megakaryocytes.

The results of monitoring the differentiation into the platelets with a flow cytometer is shown in Figure 8, and the results of monitoring the differentiation into the mature megakaryocytes with a flow cytometer is shown in Figure 9. Figure 10 represents the quantified graph based on the results of Figures 8 and 9. Therein, the platelets after the culture for differentiation are indicated by "CD41+42+Plt", whereas the mature megakaryocytes are indicated by "CD41+MK". The specific numerical values for each cell number in Figure 10 are shown in Table 5. The number of the mature megakaryocytes was increased moderately from the 0 day to the 6th day after the differentiation (Figure 9, Table 5). On the other hand, the number of the platelets was very low until the 3rd day, but was increased sharply after the 4th day (Figure 8, Table 5). On the 4th day, the number of the platelets and the number of the mature megakaryocytes were almost the same, but on the 6th day, the number ratio of the platelet was 15 whereas the number ratio of the mature megakaryocytes was 1. Table 5 shows the ratio of the number of platelets to the number of megakaryocytes calculated from the results of each flow cytometer and the graph thereof.

**Table 5**

| | Day 0 | Day 1 | Day 2 | Day 3 | Day 4 | Day 5 | Day 6 |
|---|---|---|---|---|---|---|---|
| Plt | 1456 | 1502 | 5712 | 19818 | 129800 | 740515 | 2839530 |
| MK | 48224 | 130844 | 105846 | 103917 | 121055 | 105108 | 187193 |

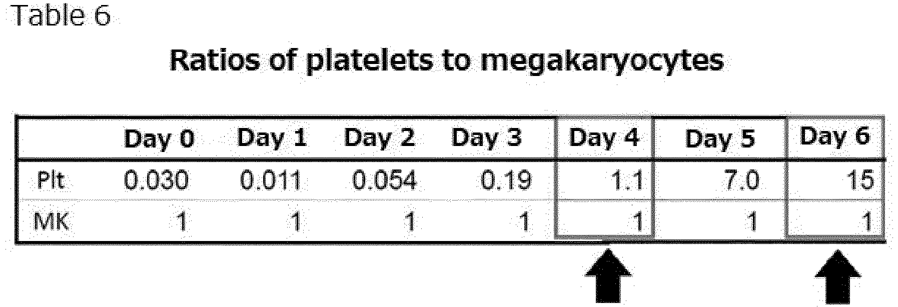

Table 6 shows that, as the ratio of the number of platelet to megakaryocyte, 2 × 10² to 1.5 × 10⁴ platelets to 1 × 10³ megakaryocytes were comprised.

These results suggest that, since the ratio of platelets/megakaryocytes is increased with the progress of the differentiations thereof, the freeze-dried preparations comprising the platelets/megakaryocytes at a various ratio can be prepared by changing the number of days for differentiations, and therefore, it is concluded that the number of days for differentiation could be adjusted in accordance with the purpose of use.

### Industrial applicability

According to the present invention, PRPs derived from platelets induced to differentiate from stem cells including iPS cells are freeze-dried, and the same are stored, thereby making it to provide a pharmaceutical medicament when necessary. As a future prospect, if FD-PRPs derived from completely antigen-free iPS cells are developed and can be safely used for allogeneic patients, the need for preparation when used in surgery would be eliminated, thus dramatically increasing in the possibility if their general usefulness as effective and new medicaments, and thereby expecting that remedies in the fields of orthopedics and dermatology would be significantly improved. In addition, the ready-made and commercialized materials may effectively promote a fast bone adhesion and a skin repair in fractures and spinal surgeries without any patient invasion or burden on the medical staff, and therefore a dramatic improvement in patient's ADL and QOL may be achieved, expecting that it would greatly contribute to the improvement of the medical economy by reducing and alleviating complications due to surgery, and shortening the length of hospital stay. Even additionally, the vigorous tissue repair potency of PRPs according to the present invention may be useful to treat ulcerative intractable wounds, nerve injury, spinal cord injury, and the like, which have been conventionally difficult to be treated, expecting that the medicaments of the present invention would develop into commercial industrial products to be spread worldwide.

## Claims

1. A freeze-dried preparation which comprises megakaryocytes and platelets.

2. The freeze-dried preparation according to claim 1, wherein the megakaryocytes and the platelets are induced to differentiate from stem cells in vitro.

3. The freeze-dried preparation according to claim 1 or 2, wherein the stem cells are pluripotent stem cells or hematopoietic stem cells.

4. The freeze-dried preparation according to claim 2 or 3, wherein the pluripotent stem cells are iPS cells.

5. The freeze-dried preparation according to any one of claims 1 to 4, which further comprises a growth factor released from the megakaryocytes and/or the platelets.

6. The freeze-dried preparation according to claim 5, wherein the growth factor is at least one factor selected from the group consisting of bone morphogenetic proteins, platelet-derived growth factors, platelet-derived angiogenesis factors, transforming growth factors, vascular endothelial growth factors, epithelial growth factors, fibroblast growth factors, hepatocyte growth factors, platelet-derived endothelial cell growth factors, insulin-like growth factors, and platelet factor IV.

7. A pharmaceutical composition for promoting a bone adhesion, or treating or preventing a skin disease, which comprises the freeze-dried preparation according to any one of claims 1 to 6.

8. The pharmaceutical composition according to claim 7, which comprises the freeze-dried preparation according to any one of claims 1 to 6 in an amount effective for promoting a bone adhesion, or treating or preventing a skin disease.

9. A method for promoting a bone adhesion, or treating or preventing a skin disease, which comprises administering the freeze-dried preparation according to any one of claims 1 to 6 to a subject in need of such treatment.

10. The method according to claim 9, which comprises administering the freeze-dried preparation according to any one of claims 1 to 6 in an amount effective for promoting a bone adhesion, or treating or preventing a skin disease to a subject in need of such treatment.

11. The freeze-dried preparation according to any one of claims 1 to 6, for promoting a bone adhesion, or treating or preventing a skin disease.

12. Use of the freeze-dried preparation according to any one of claims 1 to 6 for manufacturing a medicament for promoting a bone adhesion, or treating or preventing a skin disease.

13. A method for producing a freeze-dried preparation comprising megakaryocytes and platelets, which is **characterized in**:
(A) preparing a cell mixture of platelets and megakaryocytes, and
(B) freeze-drying the cell mixture of the platelets and the megakaryocytes.

14. The method according to claim 13, wherein the megakaryocytes are immortalized megakaryocytes that are induced to differentiate from stem cells in step (A).

15. The method according to claim 13 or 14, wherein the cell mixture prepared in step (A) is activated.
